(19) Europäisches Patentamt / European Patent Office / Office européen des brevets

(11) **EP 2 888 688 B1**

(12) **EUROPEAN PATENT SPECIFICATION**

(45) Date of publication and mention of the grant of the patent:
**04.09.2019 Bulletin 2019/36**

(21) Application number: **13742577.3**

(22) Date of filing: **19.07.2013**

(51) Int Cl.:
***G16B 20/00*** *(2019.01)*

(86) International application number:
**PCT/US2013/051331**

(87) International publication number:
**WO 2014/015273 (23.01.2014 Gazette 2014/04)**

(54) **COMPREHENSIVE FMR1 GENOTYPING**

UMFASSENDE FMR1-GENOTYPISIERUNG

GENOTYPAGE COMPLET DU FMR1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2012 US 201261674167 P**

(43) Date of publication of application:
**01.07.2015 Bulletin 2015/27**

(73) Proprietor: **Asuragen, INC.**
**Austin, TX 78744 (US)**

(72) Inventors:
- **CHOUDHARY, Ashish**
  **Austin, TX 78748 (US)**
- **LATHAM, Gary**
  **Austin, TX 78739 (US)**
- **SAH, Sachin**
  **Austin, TX 78739 (US)**

(74) Representative: **Schlich, George**
**Schlich**
**9 St Catherine's Road**
**Littlehampton, West Sussex BN17 5HS (GB)**

(56) References cited:
**WO-A1-2010/110835**

- **LIANGJING CHEN ET AL: "An Information-Rich CGG Repeat Primed PCR That Detects the Full Range of Fragile X Expanded Alleles and Minimizes the Need for Southern Blot Analysis", THE JOURNAL OF MOLECULAR DIAGNOSTICS, vol. 12, no. 5, 1 September 2010 (2010-09-01), pages 589-600, XP055087548, ISSN: 1525-1578, DOI: 10.2353/jmoldx.2010.090227**
- **L A Larsen ET AL: "Haplotype and AGG-interspersion analysis of FMR1 (CGG)(n) alleles in the Danish population: implications for multiple mutational pathways towards fragile X alleles", American journal of medical genetics, 17 July 2000 (2000-07-17), pages 99-106, XP055087218, UNITED STATES Retrieved from the Internet: URL:http://www.ncbi.nlm.nih.gov/pubmed/10869110 [retrieved on 2013-11-08]**
- **E. J. Kremer ET AL: "Mapping of DNA Instability at the Fragile X to a Trinucleotide Repeat Sequence p(CCG)n", Proc. Nad. Acid. Sci. U.SA, 1 January 1991 (1991-01-01), pages 1711-1714, XP055087236, Retrieved from the Internet: URL:http://www.sciencemag.org/content/252/5013/1711.long [retrieved on 2013-11-07]**
- **CAROLYN M. YRIGOLLEN ET AL: "The Role of AGG Interruptions in the Transcription of FMR1 Premutation Alleles", PLOS ONE, vol. 6, no. 7, 1 July 2011 (2011-07-01), page e21728, XP055087581, ISSN: 1932-6203, DOI: 10.1371/journal.pone.0021728**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **B. BODEGA: "Influence of intermediate and uninterrupted FMR1 CGG expansions in premature ovarian failure manifestation", HUMAN REPRODUCTION, vol. 21, no. 4, 16 December 2005 (2005-12-16), pages 952-957, XP055087663, ISSN: 0268-1161, DOI: 10.1093/humrep/dei432**

## Description

This application claims the benefit of priority under 35 U.S.C. § 119 to U.S. Provisional Application No. 61/674,167, filed on July 20, 2012.

## SEQUENCE LISTING

The instant application contains a Sequence Listing.

The present disclosure relates generally to an apparatus and software for automated nucleic acid analysis, particularly to methods for analyzing and genotyping regions in nucleic acid compositions, such as isolated nucleic acid products, fragments, and templates, that contain nucleotide repeats.

Genetic loci that comprise regions of nucleotide repeats (e.g., dinucleotide repeats, trinucleotide repeats, etc.) are common in the human or animal genome. Genetic loci that have enriched GC content are also common. In some circumstances, the expansion of GC-rich regions, or the expansion of nucleotide repeats, can be associated with various disease states. For example, the expansion of CGG repeats in the 5' untranslated region (UTR) of the Fragile X Mental Retardation-1 gene (*FMR1*), located on the X chromosome, is associated with Fragile X Syndrome (FXS) and related disorders and phenotypes. In most people, the trinucleotide CGG is repeated approximately 5-44 times in the 5' untranslated region (UTR) of the *FMR1* gene ("CGG repeat region"). Expansions in this region to greater than about 45 CGG repeats, and particularly to greater than about 200 CGG repeats, have been associated with FXS. FXS phenotypes may include mental retardation, autism, anxiety, and other cognitive or behavioral conditions. (J. Mol. Diag. 10(6): 496-501 (2008)). Likewise, expansion of the CCG trinucleotide repeat region ("CCG repeat region") in the 5' UTR of the *FMR2* gene is associated with X-linked intellectual disabilities, and particularly with Fragile X syndrome E (FRAXE). FRAXE is a common form of X-linked mental retardation.

Methods for genotyping GC-rich sequences and sequences comprising nucleotide repeats, such as the CGG and CCG repeats in *FMR1* and *FMR2,* include restriction enzyme digestion and polymerase chain reaction (PCR) strategies. Restriction digest analysis can provide a crude measure of the size of a triplet repeat region. However, restriction digest analysis is limited in resolution, does not easily detect short interruptions (such as single codon AGG interruptions within a CGG repeat region) and cannot determine methylation status.

PCR strategies may provide greater accuracy in reconstructing various genotypes. However, limitations exist in the amplification and sequencing of genetic loci that comprise long repeat sequences or contain GC-rich sequences that hinder the ability to reconstruct genotypes for these loci. Efforts to optimize PCR procedures for the analysis of the CGG repeats in *FMR1,* for example, have been attempted, and include modifications to conventional PCR assay conditions. (*See* Genome Res. 6(7): 633-8, (1996); J. Mol. Diag. 8: 544-550, (2006); and Am. J. Med. Genet. 51(4): 527-34, (1994)). More recently, PCR techniques have been developed that permit more reliable amplification of genomic loci having over 200 CGG or CCG repeats. *See* US Application Nos. 2010/0209970, 2010/0243451, and 2012/0107824, which describe PCR methods for sequencing GC-enriched repeat regions. These methods often provide information characterizing a repeat region, such as the length of the region, the number of repeats within the region, and the presence of any interruptions within the repeat region. However, PCR alone does not permit rapid or high throughput reconstruction of the full genotype from the parameter information characterizing the GC-rich region, especially where the region contains short interruptions, such as the single codon AGG sequences observed in the *FMR1* 5' UTR.

Recent studies have indicated that AGG interruptions within the CGG repeat region in the 5' UTR of *FMR1* may confer DNA stability and may reduce the risk of expansion in the triplet repeat region in offspring that is associated with FXS and related disorders, including Fragile X-associated tremor/ataxia syndrome (FXTAS), fragile X-related primary ovarian insufficiency (FXPOI), and dopamine-responsive Parkinsonism. (*See* Eichler et al., Nat. Genet. 8: 88-94 (1994); Nolin et al., Am. J. Hum. Genet. 72: 454-64 (2003); Yrigollen et al., Genet. Med., in press). Although it is possible to reconstruct genotypes, such as the CGG repeat region in the 5' UTR of *FMR1,* using manual interpretation, this process can be complex and time-consuming. Thus, a method for fast, accurate, and high-throughput genotyping of regions having nucleotide repeats or high GC content, such as the CGG repeat region in the 5' UTR of *FMR1* and the CCG repeat region in the 5' UTR of *FMR2,* could offer diagnostic benefits. For example, automation of data interpretation could improve workflow in the diagnostics laboratory by allowing for faster validation of assay data and by ensuring robust and accurate sequencing results.

Liangjing Chen et al, in a paper entitled "An Information-Rich CGG Repeat Primed PCR that detects the full range of fragile X expanded alleles and minimizes the need for southern blot analysis" in the Journal of Molecular Diagnosis, vol. 12, no. 5, 1 September 2010 describes a method for PCR amplification of CGG repeat regionals in the FMR1 gene using a set of three PCR primers and a capillary electrophoresis protocol. This generates data relating to the total CGG repeat length and presence of AGG interruptions. No use is made of this data.

Accordingly, disclosed herein are methods for the automated reconstruction of a genotype comprising one or more GC-rich region or one or more nucleotide repeat region.

According to the invention there is provided, a method of automated reconstruction of a genotype, comprising (a) providing a nucleic acid isolated from a patient sample, wherein the nucleic acid has at least one GC-rich nucleotide repeat region comprising one or more interruptions; (b) determining parameter information for the nucleic acid wherein determining parameter information comprises measuring the total length of the at least one GC-rich nucleotide repeat region, the distance from the start of the at least one GC-rich nucleotide repeat region to the one or more interruptions in the region, and the distance from the end of the at least one GC-rich nucleotide repeat region to the one or more interruptions in the region; and (c) using an apparatus to apply automated exhaustive enumeration to the parameter information to generate a reconstructed genotype, wherein exhaustive enumeration comprises

i) using the total length of the at least one GC-rich nucleotide repeat region and either the distance from the start of the at least one GC-rich nucleotide repeat region to the one or more interruptions in the region or the distance from the end of the at least one GC-rich nucleotide repeat region to the one or more interruptions in the region to generate a set of potential genotypes comprising all possible arrangements of the one or more interruptions in the region; and
ii) evaluating the set of potential genotypes to determine a solution genotype that satisfies all the parameter information.

In certain embodiments, the apparatus used to conduct automated exhaustive enumeration comprises a processor and a memory device communicatively coupled to the processor, wherein the memory device has stored therein machine-executable instructions that, when executed by the processor, cause the processor to receive parameter information and to conduct an exhaustive enumeration analysis. In some embodiments, the reconstructed genotype can be confirmed by manually comparing the genotype to the parameter information, by performing a restriction digest, or by sequencing the nucleic acid having at least one GC-rich nucleotide repeat region.

In various embodiments, the methods described above can be used to reconstruct a genotype when a genotype from a parent of the patient is not known.

In various embodiments, the methods described above can be used to reconstruct a genotype for the *FMR1* or *FMR2* genes or fragments thereof, or the CGG and CCG repeat regions of those genes and fragments. In these embodiments, determining the parameter information comprises determining the total length of at least one CGG or CCG repeat region, the distance from the start of the at least one CGG or CCG repeat region to one or more AGG interruptions in the region, and the distance from the end of the at least one CGG or CCG repeat region to one or more AGG interruptions in the region. In some embodiments, the parameter information is determined by polymerase chain reaction and capillary electrophoresis.

In certain embodiments, the methods described above can be used to detect a genotype or phenotype associated with an *FMR1* or *FMR2* disorder. In some embodiments, the *FMR1* or *FMR2* disorder is Fragile X Syndrome (FXS), Fragile X syndrome E (FRAXE), Fragile X-associated tremor/ataxia syndrome (FXTAS), fragile X-related primary ovarian insufficiency (FXPOI), or dopamine-responsive Parkinsonism.

In various embodiments, an apparatus is provided for the automated reconstruction of a genotype, comprising (a) a processor; and (b) a memory device communicatively coupled to the processor, the memory device having stored therein machine-executable instructions that, when executed by the processor, cause the processor to (i) receive parameter information for a nucleic acid, where the nucleic acid has at least one GC-rich nucleotide repeat region comprising one or more interruptions; and wherein the parameter information comprises the total length of the at least one GC-rich nucleotide repeat region, the distance from the start of the at least one GC-rich nucleotide repeat region to the one or more interruptions in the region, and the distance from the end of the at least one GC-rich nucleotide repeat region to the one or more interruptions in the region; (ii) generate a set of potential genotypes comprising all possible arrangements of the one or more interruptions in the region; (iii) evaluate the set of potential genotypes to produce a solution genotype that satisfies all the parameter information; and (iv) store the solution genotype on the memory device. In some embodiments, the apparatus further comprises a monitor communicatively coupled to the processor and memory device, wherein the machine-executable instructions stored on the memory device instruct the processor to display the solution genotype on the monitor. In some embodiments, the apparatus further comprises a printer communicatively coupled to the processor and memory device, wherein the machine-executable instructions stored on the memory device instruct the processor to print the solution genotype on the printer.

In various embodiments, a machine-readable medium is provided, comprising machine-executable instructions that, when executed by a processor, causes the processor to (a) receive parameter information for a nucleic acid, wherein the nucleic acid has at least one GC-rich nucleotide repeat region comprising one or more interruptions; and wherein the parameter information comprises the total length of the at least one GC-rich nucleotide repeat region, the distance from the start of the at least one GC-rich nucleotide repeat region to the one or more interruptions in the region, and the distance from the end of the at least one GC-rich nucleotide repeat region to the one or more interruptions in the region; (b) generate a set of potential genotypes comprising all possible arrangements of the one or more interruptions in the region; (c) evaluate the set of potential genotypes to produce a solution genotype that satisfies all the parameter information; and (d) store the solution genotype on a memory device. In some embodiments, the machine-executable instructions instruct the processor to display the solution genotype on a monitor. In some embodiments, the machine-executable instructions instruct the processor to print the solution genotype on a printer.

## BRIEF DESCRIPTION OF THE DRAWINGS

Fig. 1 shows an example of data from a CGG Length Assay, an Anch_A Assay, and an Anch_T assay generated from a sample where the genotype was correctly identified using exhaustive enumeration. PCR product sequence length is shown on the horizontal axis, and the number of PCR products having a particular length is shown on the vertical axis.

Fig. 2 shows an example of data from a CGG Length Assay, an Anch_A Assay, and an Anch_T assay generated from a sample where exhaustive enumeration did not produce a solution genotype. PCR product sequence length is shown on the horizontal axis, and the number of PCR products having a particular length is shown on the vertical axis.

Fig. 3 shows an example of data from a CGG Length Assay, an Anch_A Assay, and an Anch_T assay generated from a sample where exhaustive enumeration identified two potential solution genotypes. PCR product sequence length is shown on the horizontal axis, and the number of PCR products having a particular length is shown on the vertical axis.

Fig. 4A shows an example of peak height data from a CGG Length Assay plotted against peak location (solid squares) and a best fit curve of predicted peak height data based on a solution genotype generated by exhaustive enumeration that has been overlaid on the original data (open circles). Fig. 4B shows a second best fit curve based on a second solution genotype generated by exhaustive enumeration overlaid on the same data as in Fig. 4A.

## DESCRIPTION OF CERTAIN EXEMPLARY EMBODIMENTS

Reference will now be made in detail to certain exemplary embodiments according to the present disclosure, certain examples of which are illustrated in the accompanying drawings.

The section headings used herein are for organizational purposes only and are not to be construed as limiting the subject matter described.

To assist in understanding the present invention, certain terms are first defined. Additional definitions are provided throughout the application.

In this application, the use of the singular includes the plural unless specifically stated otherwise. Also in this application, the use of "or" means "and/or" unless stated otherwise. Furthermore, the use of the term "including," as well as other forms, such as "includes" and "included," are not limiting. Any range described herein will be understood to include the endpoints and all values between the endpoints.

As used herein, a "nucleic acid" is any contiguous nucleobase residues or analogs that have been removed from their natural environment and isolated from a subject and/or for which a genotype reconstruction is sought. A nucleic acid can comprise a gene, gene fragment, or genomic region isolated from a subject. As used herein, a "genotype" is the nucleobase sequence of a nucleic acid.

As used herein, "GC-richness" is the fraction or percentage of total nucleobase residues in a nucleic acid or a fragment of that nucleic acid that are guanine residues, cytosine residues, or analogs thereof. For example, a 100 nucleotide sequence that contains exactly 30 cytosines, exactly 30 guanines, exactly one cytosine analog, and exactly one guanine analog has a GC-richness of 62%. In some embodiments, a "GC-rich" nucleic acid or region of a nucleic acid is one that contains more than about 50% guanine residues, cytosine residues, or analogs thereof (e.g., more than about 50, 51, 55, 60, 65, 70, 75, 80, 85, 90, 95, 96, 97, 98, 99, or 99.5% guanine residues, cytosine residues, or analogs thereof, or any percentage in between).

As used herein, a "repeat" or "nucleotide repeat" refers to a nucleic acid or a region of a nucleic acid comprising any short sequence of 1-20 nucleobase residues in length (e.g., a dinucleotide, trinucleotide, tetranucleotide, pentanucleotide, hexanucleotide sequence, etc.) wherein the short sequence is repeated 2 or more times (e.g., 2, 3, 4, 5, 10, 15, 20, 50, 100, 200, 500, or more repeats). For example, a nucleotide repeat would encompass a region of a nucleic acid in which the short sequence CGG is repeated two or more times. A nucleic acid or a region of a nucleic acid can be both a repeat and GC rich region. For example, the nucleic acid or region of a nucleic acid can comprise di-, tri-, tetra-, penta- or hexa-nucleotide repeats of guanine residues, cytosine residues, or analogs thereof.

A nucleic acid can comprise one or more nucleotide repeats or GC-rich regions that contain one or more interruptions. As used herein, an "interruption" in a nucleic acid refers to the presence of one or more nucleobase residues or analogs in the nucleic acid that are inconsistent with the repeat pattern or, in a GC-rich region, comprise a nucleobase other than G or C (or analogs thereof). For example, a GC-rich, nucleotide repeat region could encompass a sequence comprising 40 CGG trinucleotide repeats with two AGG sequences interspersed within the 40 CGG repeats.

*FMR1* is a gene on the X chromosome that encodes the fragile X mental retardation protein (FMRP). This protein, most commonly found in the brain, is essential for normal cognitive development and female reproductive function. The 5' untranslated region (5' UTR) of the *FMR1* gene can comprise one or more regions (e.g., 1, 2, 3, 4, 5, or more regions) having CGG repeats. The presence of a region comprising 40 or more CGG repeats in the 5' UTR is thought to be associated with *FMR1*-related disorders. The 5' UTR of the *FMR1* gene can also comprise one or more AGG sequences that interrupt a CGG repeat region.

*FMR2* (AFF2) is a gene on the X chromosome that encodes the fragile X mental retardation 2 protein. The 5' UTR of the *FMR2* gene can comprise one or more regions (e.g., 1, 2, 3, 4, 5, or more regions) having CCG repeats. Expansion of a CCG (or CGG) repeat region has been associated with X-linked intellectual disabilities, and particularly with Fragile X syndrome E (FRAXE). FRAXE is a common form of X-linked mental retardation.

**I. Comprehensive genotyping**

Disclosed herein are methods for the automated reconstruction of a genotype comprising one or more nucleotide repeat regions or GC-rich regions. For example, the methods disclosed herein can be used to reconstruct a genotype from a nucleic acid or fragment thereof comprising CGG repeats and interspersed AGG interruptions, or to reconstruct a genotype comprising CCG repeats. In some embodiments, the methods can be used to reconstruct the repeat region of the *FMR1 or FMR2* gene, or fragments thereof, or the 5' UTR of *FMR1* or *FMR2,* or fragments thereof, isolated from a subject. In certain embodiments, the methods disclosed herein are used to assist in reconstructing the genotype of *FMR1,* including the CGG repeat pattern and the location and organization of AGG interruptions within the 5' UTR of *FMR1.* In other embodiments, the methods disclosed herein are used to assist in reconstructing the genotype of *FMR2,* including the CCG repeat pattern.

In some embodiments, the methods disclosed herein are used to determine the genotype of a nucleic acid or fragment thereof from a patient sample, wherein the nucleic acid has at least one repeat or GC-rich region, and wherein the related genotype from at least one of the parents of the patient is not known. In certain embodiments, the methods disclosed herein are used to determine a genotype for the CGG or CCG repeat region in *FMR1* or *FMR2* from a patient sample, wherein the related *FMR1* or *FMR2* genotype from at least one of the parents of the patient is not known.

In various embodiments, a method for the automated reconstruction of a genotype comprises providing a sample from a patient, wherein the sample contains a nucleic acid or fragment thereof having one or more repeat regions or GC-rich regions. In some embodiments, information characterizing the nucleic acid (i.e., "parameter information") is collected, such as the total length of the repeat or GC-rich region and the distance in the forward and reverse directions to any interruptions in the repeat or GC-rich region. In some embodiments, the collected information is automatically analyzed using exhaustive enumeration, using an apparatus comprising a processor programmed to conduct an automated exhaustive enumeration analysis. In some embodiments, the exhaustive enumeration reconstructs a solution genotype (i.e., a genotype that satisfies all of the parameter information). In certain embodiments, the accuracy of the solution genotype can be evaluated by manually analyzing the genotype to confirm that it comports with all of the parameter information, or by conducting any other confirmatory assay (e.g., restriction enzyme digest, Sanger sequencing, or other forms of high throughput sequencing). In some embodiments, the solution genotype can be displayed or stored electronically on a computer, or can be printed for subsequent diagnostic and therapeutic purposes.

In certain embodiments, the reconstructed genotype can be used to detect a mutation or genotype, or to diagnose or assist in diagnosing a disorder associated with a mutation in a repeat region or GC-rich region, such as an *FMR1* or *FMR2* related mutation, genotype, or disorder.

In various embodiments, parameter information characterizing a nucleic acid can be obtained using any suitable method, such as PCR or restriction digest analyses. In certain embodiments, the parameter information includes the overall length of the repeat or GC-rich region, as well as the distance from the start of the repeat or GC-rich region to a first or subsequent interruption in the forward direction and in the reverse direction. In some embodiments, an apparatus is provided, comprising a processor programmed to analyze parameter information and to reconstruct a genotype from the information. In certain embodiments, the apparatus is used to reconstruct the genotype of the nucleic acid from the information characterizing the nucleic acid. In some embodiments, the apparatus uses exhaustive enumeration to evaluate all possible genotype reconstructions based on the length of the repeat or GC-rich region and the interruptions in the forward or reverse direction to select the reconstruction that satisfies all the parameter information (e.g., the genotype that places the interruptions in the correct positions in both the forward and reverse directions). In certain embodiments, the apparatus provides a report of the reconstructed genotype that can be displayed on a screen, saved digitally for future use, or printed as a paper record.

In various embodiments, parameter information regarding a nucleic acid can be obtained using any method known in the art, so long as it includes information regarding the total length of a repeat or GC-rich region in the nucleic acid and the distance from the start ("forward direction") and end ("reverse direction") of the GC-rich region to any interruptions. In some embodiments, restriction enzymes that cleave a nucleic acid site-specifically can be used to analyze a repeat or GC-rich region and thereby generate parameter information. For example, the presence of AGG interruptions within a CGG repeat tract of *FMR1* can be detected by digesting a nucleic acid with the restriction enzyme *EciI* (New England Biolabs Inc., Ipswich, MA, USA). In other embodiments, PCR methods can be used to generate the necessary information. For example, restriction digest and/or PCR methods can be used with an *FMR1* or *FMR2* gene or fragments thereof isolated from a patient in order to determine the length of one or more CGG or CCG repeats. The methods can also be used to determine the distance in the forward and reverse directions from the first and last CGG or CCG triplets to any internal interruptions, such as AGG interruptions interspersed within a CGG repeat region in the *FMR1* gene.

In some embodiments, suitable methods for generating parameter information include polymerase chain reaction (PCR), real-time PCR (RT-PCR), nucleic acid sequence-base amplification (NASBA), ligase chain reaction, multiplex ligatable probe amplification, invader technology (Third Wave), rolling circle amplification, in vitro transcription, strand displacement amplification, transcription-mediated amplification (TMA), RNA (e.g., Eberwine) amplification, loop-mediated isothermal amplification, or any other methods that are known to one of skill in the art. For example, *FMR1* parameter information can be generated using a two-tier PCR approach with a CGG linker primer and the Human *FMR1* PCR kit (Asuragen Inc., Austin, TX, USA). *See* Tassone et al., J Mol Diagn. 10(1):43-49 (2008); Chen et al., J Mol Diagn. 12(5): 589-600 (2010); Yrigollen et al., PLoS One 6(7): e21728 (2011). For example, a nucleic acid comprising at least one GC-rich region can be analyzed by (a) providing at least two PCR primers, including a first primer comprising CGG, CCG, GCG, CGC, GCC, or GGC repeats, and a second primer that anneals to a position outside of the GC-rich region; (b) performing PCR on the nucleic acid with the at least two different primers, wherein the PCR produces a set of PCR products; (c) resolving the set of PCR products with a high resolution technique (such as capillary electrophoresis) to generate a representation of PCR product size and abundance; and (d) deriving from the PCR product size and abundance information the length of the GC-rich region and whether or where within the GC-rich region an interruption is located.

In certain embodiments, an *FMR1* genotype is reconstructed, and some or all of the *FMR1* parameter information (e.g., the CGG repeat length and/or the distance in the forward and/or reverse directions to any AGG interruptions) can be generated using forward and reverse PCR primers comprising CGG and GCC repeats, respectively. In certain embodiments, *FMR1* parameter information can be generated using a forward primer comprising the sequence $(CGG)_5A$ (SEQ ID NO: 42) and/or a reverse primer comprising the sequence $TCC(GCC)_4$ (SEQ ID NO: 46). In some embodiments, some or all of the *FMR1* parameter information (e.g., the CGG repeat length and/or the distance in the forward and/or reverse directions to any AGG interruptions) can be generated using alternative primers. For example, primers having CGG or GCC repeat sequences that are shorter or longer than the $(CGG)_5A$ (SEQ ID NO: 42) and $TCC(GCC)_4$ (SEQ ID NO: 46) sequences mentioned above, or other sequences that are specific for the region at or near an AGG interruption, can be used.

In some embodiments, *FMR1* parameter information can be generated using a forward PCR primer comprising the sequence $(CGG)_5A$ (SEQ ID NO: 42) and/or a reverse PCR primer comprising the sequence $TCC(GCC)_4$ (SEQ ID NO: 46) to identify the location of any AGG interruptions in the CGG repeat pattern of an *FMR1* allele, while the total length of the CGG repeat can be determined using a first PCR primer comprising CGG or GCC repeats, and a second PCR primer that anneals to a position outside of the CGG repeat region. For example, the parameter information can be generated using a Human *FMR1* PCR kit (Asuragen Inc., Austin, TX, USA). In some embodiments, the primers can be used to PCR amplify segments of the *FMR1* locus, and amplitude information (i.e., the number of copies of a segment of the FMR1 locus of a particular length produced by the PCR reaction) can be measured using capillary electrophoresis. See, e.g., the methods of US Application Nos. 2010/0209970, 2010/0243451, and 2012/0107824.

In various embodiments, PCR-amplified nucleic acids are analyzed to generate parameter information, for example using capillary electrophoresis (CE) instruments familiar to those skilled in the art, such as ABI model 3100, 3130, 3730, or 3500 CE instruments (Applied Biosystems, Carlsbad, CA). Other implementations, include any instrument capable of electrophoretically or otherwise sizing and/or sequencing an amplified nucleic acid, can also be used. Exemplary PCR and analysis methods for use with repeat or GC-rich regions, such as the CGG and CCG repeat loci in the 5' UTRs of *FMR1* and *FMR2,* are described in US Application Nos. 2010/0243451 and 2012/0107824 and US Pat. No. 8,409,805. Any other method of collecting parameter information can also be used (e.g., Sanger sequencing or other forms of high throughput sequencing).

For example, parameter information characterizing the CGG and CCG repeat loci in the 5' UTRs of *FMR1* and *FMR2* or fragments thereof can be generated using the methods described in U.S. Patent Application No. 2010/0243451, including the primers, polymerase, reagents, and reaction conditions disclosed at paragraphs [0040]-[0051], [0056]-[0060], [0065]-[0067], [0089], [0094], and [0104].

For example, in some embodiments *FMR1* and *FMR2* parameter information can be generated using a primer that anneals outside of a repeat region and a primer that anneals to repeat sequences, sequence permutations, or reverse complements of the sequences (GCG, CCG, CGC, GCC, or GGC). The primers that can anneal outside of (upstream or downstream) of the repeat region may be forward or reverse primers. The primers may anneal to sequences flanking the repeat region. Examples of forward primers include CGG TGG AGG GCC GCC TCT GAG C (SEQ ID NO: 1), CAG GCG CTC AGC TCC GTT TCG GTT T (SEQ ID NO: 2), CAG TCA GGC GCT CAG CTC CGT TTC G (SEQ ID NO: 3), TCC GGT GGA GGG CCG CCT CTG AGC (SEQ ID NO: 4), GGT TCG GCC TCA GTC AGG CGC TCA GCT CCG TTT CG (SEQ ID NO: 5), GGG TTC GGC CTC AGT CAG GCG CTC AGC TCC GTT TCG (SEQ ID NO: 6), GCG GGC CGG GGG TTC GGC CTC AGT CA (SEQ ID NO: 7), CAG CGG GCC GGG GGT TCG GCC TCA G (SEQ ID NO: 8), GCA GCG GGC CGG GGG TTC GGC CTC A (SEQ ID NO: 9), GGG CCG GGG GTT CGG CCT CAG TCA G (SEQ ID NO: 10), GGG GTT CGG CCT CAG TCA GGC GCT CA (SEQ ID NO: 11), GGG GTT CGG CCT CAG TCA GGC GCT CAG (SEQ ID NO: 12), GGC GCT CAG CTC CGT TTC GGT TTC ACT TCC (SEQ ID NO: 13), TCA GGC GCT CAG CTC CGT TTC GGT TTC A (SEQ ID NO: 14), CAC TTC CGG TGG AGG GCC GCC TCT GA (SEQ ID NO: 15), TTC CGG TGG AGG GCC GCC TCT GAG C (SEQ ID NO: 16), and TCA GGC GCT CAG CTC CGT TTC GGT TTC ACG GCG GCG GCG GCG GA (SEQ ID NO: 44). Examples of reverse primers include CGC ACT TCC ACC ACC AGC TCC TCC A (SEQ ID NO: 17), GGA GCC CGC CCC CGA GAG GTG (SEQ ID NO: 18), GGG AGC CCG CCC CCG AGA GGT (SEQ ID NO: 19), CGC ACT TCC ACC ACC AGC TCC TCC AT (SEQ ID NO: 20), CGG GAG CCC GCC CCC GAG AGG TG (SEQ ID NO: 21), CCG GGA GCC CGC CCC CGA GAG GT (SEQ ID NO: 22), CCG GGA GCC CGC CCC CGA GAG GTG (SEQ ID NO: 23), CGC CGG GAG CCC GCC CCC GAG AGG TG (SEQ ID NO: 24), GCG CCG GGA GCC CGC CCC CGA GAG GT (SEQ ID NO: 25), CGC CGG GAG CCC GCC CCC GAG AGG T (SEQ ID NO: 26), GCG CCA TTG GAG CCC CGC ACT TCC ACC A (SEQ ID NO: 27), GCG CCA TTG GAG CCC CGC ACT TCC A (SEQ ID NO: 28), AGC GCC ATT GGA GCC CCG CAC TTC C (SEQ ID NO: 29), CGC CAT TGG AGC CCC GCA CTT CCA C (SEQ ID NO: 30), TTG GAG CCC CGC ACT TCC ACC ACC A (SEQ ID NO: 31), AGC CCC GCA CTT CCA CCA CCA GCT CCT C (SEQ ID NO: 32), GAG CCC CGC ACT TCC ACC ACC AGC TCC T (SEQ ID NO: 33), CAT TGG AGC CCC GCA CTT CCA CCA CCA G (SEQ ID NO: 34), CCC GCA CTT CCA CCA CCA GCT CCT CCA TCT (SEQ ID NO: 35), TAG AAA GCG CCA TTG GAG CCC CGC ACT TCC (SEQ ID NO: 36), AAG CGC CAT TGG AGC CCC GCA CTT CC (SEQ ID NO: 37), AAG CGC CAT TGG AGC CCC GCA CTT CCC CGC CGC CGC CGC CG (SEQ ID NO: 43), and AAG CGC CAT TGG AGC CCC GCA CTT CCC CGC CGC CGC CGC CT (SEQ ID NO: 45).

in some embodiments *FMR1* and *FMR2* parameter information can be generated using the primers TCAGGCGCTCAGCTCCGTTTCGGTTTCACTTCCGGT (SEQ ID NO: 38), AGCGTCTACTGTCTCGGCACTTGCCCGCCGCCGCCG (SEQ ID NO: 39), TCA GGC GCT CAG CTC CGT TTC GGT TTC A (SEQ ID NO: 40), and TCAGGCGCTCAGCTCCGTTTCGGTTTCA CGGCGGCGGCGGCGG (SEQ ID NO: 41). The methods can additionally involve using primers comprising the sequence of any of SEQ ID NOs 1-38 or 40 and comprising additional repeats of CGG or the permutations and reverse complements thereof (e.g., GCG, CCG, CGC, GCC, or GGC) appended to the 3' end. In some embodiments, the number of CGG repeats or permutations in the primer is four or five. In some embodiments, the primer contains a sequence of CGG repeats (or permutations thereof) stretching for 12-15 nucleotides or more. In some embodiments, the primer contains sequence of CGG repeats (or permutations thereof) ranging from 3 to 10 repeats. The primer may contain 3, 4, 5, 6, 7, 8, 9, or 10 repeats, and optionally an additional partial repeat of 1 or 2 C and/or G residues. Additional primers can be provided, for example, to ensure binding at a polymorphic site, or to amplify a region of known size in order to serve as an internal standard.

In some embodiments, the primer that anneals to the repeat or GC rich region has a preferential binding activity for sites in the region comprising an interruptor element. Preferential binding to site of an interruptor element can result in selective amplification of at least one product comprising the interruptor element, e.g., by using the primer in a PCR reaction with an oppositely oriented second primer that binds outside of the repeat or GC rich region. Preferential binding activity can be specific, for example, for sites comprising CGG and AGG elements, or the permutations and/or reverse complements thereof, such as a site comprising (1) one AGG element or a part of an AGG element comprising an A, and (2) three, four, five, or six CGG elements and optionally an additional partial CGG element.

In some embodiments, the primer that anneals to repeat or GC rich region and binds preferentially to a site or sites comprising an interruptor element may comprise an A, T, or U residue within or at the end of the part of the primer that anneals to repeat or GC rich sequences. For example, the primer can have an A, T, or U among or at the end of a stretch of CGG, CCG, GCG, CGC, GCC, or GGC repeats; see, for example, SEQ ID NOs 44 and 45 above. The A, T, or U residue can occur at the 3' end of the primer. When the A, T, or U residue occurs at the end of the CGG, CCG, GCG, CGC, or GCC, GGC repeats, there may or may not be a partial CGG, CCG, GCG, CGC, GCC, or GGC repeat between the A, T, or U residue and the last complete CGG, CCG, GCG, CGC, GGC, or GGC repeat. It is possible to substitute unnatural nucleotide residues that preferentially base pair with T/U or A residues relative to other natural nucleotide residues for the A, T, or U residue. Likewise, it is also possible to substitute one or more unnatural nucleotide residues that preferentially base pair with C or G residues relative to other natural nucleotide residues for one or more G and/or C residues that make up the CGG, CCG, GCG, CGC, GCC, or GGC repeats. The presence of one or more such unnatural residues within a sequence otherwise made up of CGG, CCG, GCG, CGC, GCC, or GGC repeats (optionally with an A, T, U, or corresponding unnatural residue as discussed above) does not negate the identity of said sequence within the context of the present disclosure as a sequence of CGG, CCG, GCG, CGC, GCC, or GGC repeats.

Unnatural nucleotide residues are nucleotide residues comprising a nucleobase other than adenine, thymine, guanine, cytosine, and uracil (A, T, G, C, and U, respectively). Examples of unnatural nucleotide residues that preferentially base pair with A or T/U residues include, without limitation, adducts of T, U, or A residues that preferentially base pair with A or T/U residues relative to other natural residues (e.g., 5-substituted uracil analogs); and residues comprising nucleobases such as, for example, pseudouracil and diaminopurine.

In some embodiments, a first primer is used when generating parameter information that has a preferential binding activity for sites in the repeat or GC rich region that do not comprise interruptor elements. The presence of an interruptor element can be signaled in the results of this method by a relatively low level of products whose synthesis would involved extension of the first primer bound to sites comprising the interruptor element. These low levels can appear as a gap or set of low peaks surrounded by higher peaks in an electropherogram. In some embodiments, a first primer is provided that has a preferential binding activity for sites in the repeat or GC rich region that comprise interruptor elements. The presence of an interruptor element is signaled in an anchored assay by a relatively high level of products whose synthesis involved extension of the first primer bound to sites comprising the interruptor element. The high level can appear as a spike surrounded by lower peaks and/or baseline signal in an electropherogram.

The methods of of generating parameter information may relate to amplification reactions comprising providing at least two or at least three different primers. In some embodiments, at least three different primers are provided and one of the primers is a primer that preferentially binds outside the repeat or GC rich region, a second primer preferentially binds within the repeat or GC rich region, and the third primer is a subsequence of either the first or second primer. In some embodiments, one primer is a chimeric primer comprising CGG repeats and a 5' flap sequence, and another primer has the sequence of the 5' flap sequence of the chimeric primer. It should be noted that the primer having the sequence of the 5' flap sequence of the chimeric primer can, but does not necessarily, have the entire non-repeat sequence of the chimeric primer. In other words, the sequence of part or all of one primer can be comprised by the sequence of another primer; for example, the chimeric primer comprises a 5' flap sequence, and another primer can comprise the sequence of part or all of the 5' flap. In some embodiments, the primer contains 12-15 nucleotides of a CGG repeat sequence. The 5' flap sequence may correspond to a sequence adjacent to or near to the CGG repeat region, or it may be unrelated to sequences in and around the CGG repeat region. In some embodiments, the length of the chimeric primer may be approximately 35, 40, 45, 50, or 55 nucleotdies. In some embodiments, one or more of the primers has a melting temperature ranging from 60°C to 75°C, for example, approximately 60°C, 65°C, 70°C, or 75°C.

In some embodiments, at least three different primers are provided and one primer is provided at a concentration lower than the concentration of another primer. For example, the chimeric primer is optionally provided at a lower concentration than the primer with the sequence of the 5' flap sequence of the chimeric primer. The ratio of concentrations, expressed as a fold difference, may range from 2 to 10,000 or more, for example, 10, 20, 50, 100, 200, 500, 1,000, 2,000, 5,000, or 10,000 (or any value in between). In such embodiments, the primer present at a lower concentration can be depleted in early rounds of the amplification reaction, such that extension is generally all, or nearly all, from the primers still present (which were initially present at relatively higher concentrations).

In some embodiments, the methods of generating parameter information comprise providing dNTPs in a GC/AT ratio greater than one, and at a total dNTP concentration conducive to synthesis of DNA comprising repeat or GC-rich templates. *See* U.S. Application No. 12/371,306. The GC/AT ratio may be about 1.1, 1.2, 1.4, 1.6, 2, 2.5, 3, 4, 5, 6, 7, 8, 9, 10, 11, 12, 13, 14, 15, 16, 17, 18, 19, 20, 25, or higher. The GC/AT ratio may be between 1.1 and 20, 1.1 and 15, 1.1 and 10, 1.1 and 8, 1 and 15, 1.1 and 7, 1.1 and 6, 1.1 and 5, 1.2 and 25, 1.4 and 25, 1.6 and 25, 2 and 25, 3 and 25, 4 and 25, 5 and 25, 2 and 15, 2.5 and 10, or 4 and 10. The total dNTP concentration may be about 0.4, 0.5, 0.6, 0.7, 0.8, 0.9, 1, 1.2, 1.5, 2, or 3 mM. The dNTP concentration may be between 0.4 and 3 mM, 0.5 and 3 mM, 0.6 and 3 mM, 0.7 and 3 mM, 0.8 and 3 mM, 0.9 and 3 mM, 1 and 3 mM, 0.4 and 2 mM, 0.4 and 1.5 mM, 0.4 and 1.2 mM, 0.4 and 1 mM, 0.4 and 0.9 mM, 0.4 and 0.8 mM, 0.4 and 0.7 mM, 0.5 and 2 mM, 0.5 and 1 mM, or 0.6 and 0.9 mM. "GC/AT Ratio" means the ratio of the concentration of the sum of dCTP, dGTP, and all nucleotide analogs thereof, to the concentration of the sum of dATP, dTTP, dUTP, and all nucleotide analogs thereof, in a given solution or mixture. "dNTP" stands for deoxynucleotide triphosphate and refers to dATP, dCTP, dGTP, dTTP, dUTP, and analogs thereof. "Nucleotide analogs" are molecules or ions comprising a base moiety other than the natural bases adenine (A), cytosine (C), guanine (G), thymine (T), or uracil (U), a sugar moiety identical or similar to deoxyribose, and at least one phosphate or multiple phosphate (e.g., diphosphate or triphosphate) moiety. The nucleotide analog is an analog of a specific nucleotide, in particular dATP, dCTP, dGTP, dTTP, or dUTP, when it comprises a triphosphate and a sugar moiety, the structure and configuration of both of which are suitable for incorporation into a nucleic acid double helix by a polymerase, and a base whose base pairing properties in a nucleic acid double helix and loci of incorporation by DNA polymerases in a nucleic acid double helix are most similar to one of the five previously listed nucleotides, with the exception that analogs of dTTP will generally also be analogs of dUTP and vice versa. The term "analog" used in conjunction with terms including but not limited to "nucleoside", "base", "nucleobase", or "residue" is to be interpreted in the same manner as if it were used in conjunction with "nucleotide."

In some embodiments, the methods of generating parameter information can further comprise providing buffers for the PCR amplification reactions. The buffers may comprise, for example and without limitation, tris(hydroxymethyl)aminomethane (Tris), bis-tris propane, bicarbonate, phosphate, glycine, histidine, 4-(2-hydroxyethyl)-1-piperazineethanesulfonic acid (HEPES), 3-(N-morpholino)propanesulfonic acid (MOPS), and various conjugate bases/acids and salts thereof.

In some embodiments, the methods of generating parameter information can comprise providing at least one DNA polymerase to synthesize DNA from dNTPs in a template dependent manner. The DNA polymerase may comprise a wild-type, modified, thermophilic, chimeric, engineered, and/or a mixture of more than one polymerase. The DNA polymerase may comprise Exact Polymerase (5 PRIME GmbH), AccuSure™ DNA Polymerase (Bioline), Phusion™ AccuPrime™ Pfx (Invitrogen), Platinum Taq DNA Polymerase High Fidelity (Invitrogen), Phire™ Hot Start DNA Polymerase (New England Biolabs), Phusion® Hot Start High-Fidelity DNA Polymerase (New England Biolabs), JumpStart™ REDTaq™ DNA Polymerase (Sigma-Aldrich), PfuUltra™ Hotstart DNA Polymerase (Stratagene), PfuTurbo® Cx Hotstart DNA Polymerase (Stratagene), PrimeSTAR™ HS DNA Polymerase (Takara), Extensor Hi-Fidelity PCR Enzyme (ABgene), ACCUZYME™ DNA Polymerase (Bioline), SAHARA™ DNA Polymerase (Bioline), VELOCITY DNA Polymerase (Bioline), GeneChoice® AccuPOL™ DNA Polymerase (GeneChoice, Inc.), GeneChoice® UniPOL™ DNA Polymerase (GeneChoice, Inc.), Elongase Enzyme Mix (Invitrogen), Pfx50™ DNA Polymerase (Invitrogen), Phusion DNA Polymerase (New England Biolabs), KOD HiFi DNA Polymerase (Novagen), KOD XL DNA Polymerase (Novagen), Expand 20 kb PLUS Thermostable DNA polymerase mixture (Roche Applied Science), Expand High Fidelity PLUS Thermostable DNA polymerase mixture (Roche Applied Science), Expand High Fidelity Thermostable DNA polymerase mixture (Roche Applied Science), Expand Long Template Thermostable DNA polymerase mixture (Roche Applied Science), Easy-A™ High-Fidelity PCR Cloning Enzyme (Stratagene), EXL™ DNA Polymerase (Stratagene), Herculase® Enhanced DNA Polymerase (Stratagene), Herculase® II Fusion DNA Polymerase (Stratagene), Kapa LongRange™ DNA Polymerase (Kapa Biosystems), Kapa HiFi™ DNA Polymerase (Kapa Biosystems), Kapa2G™ Robust DNA Polymerase (Kapa Biosystems), Kapa2G™ Robust HotStart DNA Polymerase (Kapa Biosystems), Kapa2G™ Fast DNA Polymerase (Kapa Biosystems), Kapa2G™ Fast HotStart DNA Polymerase (Kapa Biosystems), LA TAQ DNA Polymerase (Takara), Optimase DNA Polymerase (Transgenomic, Inc.), Exo- Pfu DNA Polymerase (Stratagene), HotMaster Taq DNA Polymerase (5 PRIME GmbH), HotTaq DNA Polymerase (Abnova Corporation), AmpliTaq Gold® DNA Polymerase (Applied Biosystems), Bst DNA Polymerase Lg Frag (New England Biolabs), MasterAmp™ Tfl DNA Polymerase (EPICENTRE Biotechnologies), Red Hot DNA Polymerase (ABgene), Thermoprime Plus DNA Polymerase (ABgene), Taq-red DNA Polymerase (AppliChem GmbH), BIO-X-ACT™ Long DNA Polymerase (Bioline), BIO-X-ACT™ Short DNA Polymerase (Bioline), Bioline HybriPol™ DNA Polymerase (Bioline), BioTherm Taq DNA Polymerase (eEnzyme LLC), EU-Taq DNA Polymerase (eEnzyme LLC), Synergy Taq DNA Polymerase (eEnzyme LLC), GeneChoice® RedPOL™ DNA Polymerase (GeneChoice, Inc.), AccuPrime™ GC-Rich DNA Polymerase (Invitrogen), PyroPhage® 3173 DNA Polymerase, Exo Minus (Lucigen), 9 Degrees North (Modified) DNA Polymerase (New England Biolabs), Therminator DNA Polymerase (New England Biolabs), Pwo DNA Polymerase (Roche Applied Science), Paq5000™ DNA Polymerase (Stratagene), YieldAce™ DNA Polymerase (Stratagene), e2TAK™ DNA Polymerase (Takara), or naturally occurring DNA polymerases from *P. kodakaraensis, P. furiosus, T. gorgonarius, T. zilligii, T. litoralis "VentTM", P. GB-D "Deep Vent", T. 9N-7, T. aggregans, T. barossii, T. fumicolans, T. celer, Pyrococcus sp. strain ST700, T. pacificus, P. abysii, T. profundus, T. siculi, T. hydrothermalis, Thermococcus sp. strain GE8, T. thioreducens, P. horikoshii or T. onnurineus NA1, Thermococcus sp. 9°N-7, Thermococcus sp. GI-J, Thermococcus sp. MAR-13, Thermococcus sp. GB-C, Thermococcus sp. GI-H, Thermus aquaticus, Thermus thermophilus, Thermus caldophilus, Thermus filiformis, Thermus flavus, Thermotoga maritima, Bacillus stearothermophilus,* or *Bacillus caldotenax.*

In some embodiments, at least one of the primers comprises a radiologically or electromagnetically detectable moiety. Radiologically detectable moieties include radioactive isotopes that emit detectable particles, such as beta or gamma particles, for example, $^{14}C$, $^{3}H$, $^{32}P$, $^{33}P$, $^{35}S$, and $^{125}I$. Electromagnetically detectable moieties include chemical entities that interact with electromagnetic radiation (including absorbance, emission, or both) in a detectable way, such as chromophores and fluorophores, for example, fluorescein, FAM, cyanine dyes, rhodamine dyes, etc.

In some embodiments the method of generating parameter information can comprise performing at least two assays to generate parameter information, wherein each assay comprises (a) providing primers, wherein the primers of the at least two assays are non-identical; (b) performing an amplification reaction to produce a set of amplified products; (c) resolving the set of products, at least two representations of product size and abundance being produced from the at least two assays; and (d) deriving information about whether an interruptor sequence is present in the at least one CGG-rich region or where within the at least one CGG-rich region an interruptor sequence is located. In some embodiments, the at least two assays use oppositely oriented primers comprising CGG, CCG, GCG, CGC, GCC, or GGC repeats. That is, a first assay can comprise providing primers comprising a primer comprising repeats that is oriented upstream, and the second assay can comprise providing primers comprising a primer comprising repeats that is oriented downstream, or vice versa. In some embodiments, samples which comprise at least two different alleles of the locus comprising the CGG-rich region (due to being at least one of heterozygous, aneuploid, or mosaic for that locus) can be analyzed to determine at least one detail regarding the genotype.

In various embodiments, the methods for generating parameter information can be used to analyze a template comprising GC-rich repeat sequences, such as, for example, CGG or CCG trinucleotides. This analyzing can comprise performing an amplification reaction and resolving the structures of the amplified products at high resolution. The high resolution may be a resolution sufficient to distinguish amplified products containing, for example, 20 versus 21, or 20 versus 22, 20 versus 23, 20 versus 24, or 20 versus 25 trinucleotide repeats, or in some embodiments, amplified products differing in length by 1, 2, 3, 4, 5, 6, 7, 8, 9, 10, 11, 12, 13, 14, 15, 16, 17, or 18 nucleotides or base pairs. Examples of techniques that may be employed in the methods to accomplish the resolving step include, without limitation, capillary electrophoresis, and polyacrylamide gel electrophoresis (PAGE; some embodiments of PAGE being commonly referred to in the art as "running a sequencing gel"), or "lab-on-a-chip" type microfluidics/electrophoresis systems. Instrumentation for performing capillary electrophoresis may be obtained from, for example, vendors such as Applied Biosystems (ABI), Beckman, Agilent, and Qiagen, and suitable instruments include without limitation ABI 310, 3100, 3130/3130xl, or 3730/3730xl; Beckman P/ACE MDQ; Agilent 2100 Bioanalyzer; and Qiagen QIAxcel. The process of resolving the amplified products electrophoretically can involve the use of liquid polymers, including, for example, POP-7, POP-6, POP-5, or POP-4, all of which are sold by Applied Biosystems. In some embodiments, a polymer that can resolve amplified products containing approximately 250, 300, 350, 400 or more trinucleotide repeats precisely according to size, such as, for example, POP-4, is used in resolving the products. Resolving the set of amplified products at high resolution can be accomplished using a machine, for example, chosen from machines that comprise a source of voltage (e.g., a DC power supply), machines that comprise a source of pressure (e.g., a pump), and machines that comprise a column or capillary suitable for performing chemical separations. The machine can comprise more than one of the foregoing components.

In some embodiments, resolving the amplified products at high resolution leads to the production of a representation of product size and abundance. This representation may be an image or graph that one of skill in the art can interpret, visually or with the aid of instrumentation such as, for example, a computer with appropriate software or a densitometer, to understand the size(s) and amount(s) of the products of the reaction. In some embodiments, the representation is an electropherogram, photograph, graph, plot, or autoradiogram. The representation may be derived or recorded from photons or beta particles emitted by the products or dye molecules bound to the products; these may be detected, for example, photographically or electronically, and processed or developed to generate the representation.

In some embodiments, the methods for generating parameter information comprise deriving information about CGG repeat number (i.e., how many CGG repeats, or trinucleotide repeats generally, are within a template) from the representations of product size and abundance. Such derivation may comprise counting the number of species of amplified products observable in the representation to determine the repeat number (starting from the number of repeats comprised by the smallest product, which may, for example, be 4 or 5), or estimating the repeat number based on the position of the largest amplified product observable in the representation. In some embodiments, the CGG-rich region is comprised by the 5' UTR of FMR1 or the 5' UTR of FMR2.

In some embodiments, the methods for generating parameter information comprise detecting whether interruptor elements are present within a CGG-rich region. In some embodiments, the interruptor elements are AGG trinucleotides. Detecting whether these are present may be achieved, for example, by determining positions in the template where binding of the first primer was substantially reduced, or by determining a set of lengths at which the amount of product is substantially reduced compared to neighboring lengths. For example, if the $10^{th}$ trinucleotide was AGG in a region with at least 14 total trinucleotide repeats and an amplification reaction was performed involving use of a primer comprising 4 CGG repeats, the products with 10, 11, 12, and 13 CGG repeats would be expected to be present in a substantially reduced amount relative to the neighboring products with 9 and 14 repeats. The degree to which the amount is reduced can range from 25% to 95% or more, for example, 25%, 30%, 35%, 40%, 45%, 50%, 55%, 60%, 65%, 70%, 75%, 80%, 85%, 90%, or 95%. The degree of reduction will generally depend on the zygosity of the sample; for example, a sample wherein the CGG-rich region is from an individual heterozygous for an allele comprising the CGG-rich region, or for a specific AGG repeat, may show a reduction in the amount of the corresponding products ranging from 25% to 75%. A sample wherein the CGG-rich region is from an individual hemizygous or homozygous for an allele comprising the CGG-rich region, or for a specific AGG repeat, may show a reduction in the amount of the corresponding products ranging from 50% to 95% or more.

Once the parameter information characterizing a repeat or GC-rich region has been collected, it is theoretically possible to assemble a genotype using manual interpretation. However, this manual interpretation can be a slow and complex process, particularly when the gene of interest comprises more than one repeat or GC-rich region and/or more than one interruption. For example, an *FMR1* gene or fragment thereof comprising two CGG repeat regions and two AGG interruptions would require the evaluation of sixteen potential solutions before the correct genotype could be identified (see below for an explanation of the sixteen possible combinations). Accordingly, in various embodiments, a method for automated genotype reconstruction using exhaustive enumeration is provided. In some embodiments, the use of exhaustive enumeration increases the accuracy and/or speed of genotype reconstruction.

In various embodiments, exhaustive enumeration comprises: (a) providing parameter information regarding the length of one or more repeat or GC-rich regions in a nucleic acid and the number of interruptions within those regions, (b) using the information regarding the length of a repeat or GC-rich region and the distance from the start of the region to each interruption (in either the forward or reverse direction) to generate a set of potential genotypes comprising all possible arrangements of the interruptions, and (c) evaluating the set of potential genotypes to determine the solution genotype that satisfies all the parameter information. In the embodiments where the interruptions in the forward direction are used to construct the possible genotypes, each potential genotype in the set is then evaluated using the information regarding the interruptions in the reverse direction. In the embodiments where the interruptions in the reverse direction are used to construct the possible genotypes, each potential genotype in the set is then evaluated using the information regarding the interruptions in the forward direction. In certain embodiments, the potential genotype that matches the forward and reverse interruption information is selected as the solution genotype.

In an embodiment, the exhaustive enumeration analysis can be represented by formula, $\pi|C_i|$, which indicates the product of the number of potential genotypes for the possible alleles, where $|C_i|$ is the number of candidate genotypes for allele i. As a result, this formula provides the number of possible genotypes that can be reconstructed from the parameter information (i.e., the set of potential genotypes that can be recreated using the length of the repeat or GC-rich region and the number of interruptions within the region in either the forward or reverse directions). In an embodiment, each of the $C_i$ potential genotypes generated using the forward interruption information is individually evaluated using the reverse interruption information to select a solution genotype. In another embodiment, each of the $C_i$ potential genotypes generated using the reverse interruption information is individually evaluated using the forward interruption information to select a solution genotype.

The exhaustive enumeration method can be conducted using an apparatus comprising a processor (e.g., a computer) programmed to conduct exhaustive enumeration analysis. In some embodiments, the processor is programmed to receive information regarding a nucleic acid and then apply exhaustive enumeration to reconstruct a solution genotype for the nucleic acid. In some embodiments, the apparatus also comprises a monitor to display input information and/or the solution genotype. In some embodiments, the solution genotype is stored electronically on the apparatus, and/or is capable of being printed for further diagnostic or therapeutic uses.

As described in more detail in the examples below, the exhaustive enumeration method can be used, in some embodiments, to reconstruct a genotype of the CGG repeat region in the *FMR1* gene. The 5' UTR of *FMR1* can comprise one or more CGG repeat regions, each of which may contain one or more AGG interruptions within the region. Where more than one AGG interruption is present, these generally do not occur contiguously (i.e., it is rare to find a CGG repeat region comprising $(AGG)_n$, where n is greater than or equal to 2). Various techniques for analyzing the *FMR1* gene or fragments thereof, such as the PCR methods described in US Application Nos. 2010/0209970, 2010/0243451, and 2012/0107824, often yield parameter information regarding the length of the CGG repeat region and the distance in the forward and reverse directions to each AGG interruption within the CGG repeat region. In various embodiments, this *FMR1* parameter information can be used to generate a list of all potential genotypes having the correct CGG repeat length and the correct AGG interruptions in either the forward or reverse direction. Using exhaustive enumeration, each potential solution can then be evaluated for consistency in the forward and reverse directions, and a solution genotype displaying the correct position(s) for the AGG interruption(s) within the CGG repeat sequence can be selected that satisfies both the forward and reverse distance requirements, and also does not place any AGG interruption proximal to another AGG interruption.

In certain embodiments, exhaustive enumeration may produce more than one solution genotype when reconstructing the genotype. The identified solution genotypes may be further analyzed. For example, exhaustive enumeration may generate more than one *FMR1* genotype displaying the correct position(s) for the AGG interruption(s) within the CGG repeat sequence and satisfying both the forward and reverse distance requirements, and also not placing any AGG interruption proximal to another AGG interruption. In some embodiments, when multiple solution genotypes are identified by exhaustive enumeration, the genotypes can be evaluated manually against the parameter information to select a single solution genotype that best matches the data. For instance, *FMR1* solution genotypes can be evaluated by manually comparing the genotypes against the peak height and pattern of peaks reported by capillary electropherogram in a CGG length assay (see, e.g., the CGG length assay data in Figs. 3 and 4). In some embodiments, the genotype that best fits the peak height and pattern of peaks generated in the CGG length assay can be selected as the single solution genotype.

In some embodiments, when exhaustive enumeration reconstructs the genotype by producing more than one solution genotype, the exhaustive enumeration method can further contain additional steps designed to automate the analysis of the genotypes to select a single solution. In some embodiments, instructions on machine-readable media (e.g., exhaustive enumeration software) can be programmed to generate predictive parameter data based on each solution genotype, fit that information to some or all of the original parameter data, and select the genotype where the predictive data best fits the original data (i.e., the genotype that minimizes the sum of the error between the predictive and original data). For example, when exhaustive enumeration generates more than one *FMR1* solution genotype, the software can generate predictive CGG length assay data based on each genotype, compare that predictive data to the actual CGG length assay data, and select the genotype that minimizes the sum of the error between the two data sets. An example using this method to fit predictive data from two different *FMR1* solution genotypes identified by exhaustive enumeration is shown in Fig. 4A-4B. In the example shown in Fig. 4A-4B, the software would select the genotype modeled in Fig. 4A as the single solution genotype because it reduces the sum of the error, as compared to the error in Fig. 4B.

In some embodiments, once a solution genotype is identified using exhaustive enumeration, the solution can be further evaluated by manual comparison to confirm that it satisfies all of the parameter information characterizing the nucleic acid (such as the total length of the repeat or GC-rich region and the distance in the forward and reverse directions to any interruptions), or by conducting any other confirmatory assay known to one of skill in the art (e.g., restriction enzyme digest, Sanger sequencing, or high throughput sequencing). For example, the presence of AGG interruptions within a CGG repeat tract of *FMR1* can be confirmed by digesting the nucleic acid sample with the restriction enzyme *EciI* (New England Biolabs Inc., Ipswich, MA, USA).

## II. SAMPLES

The methods provided herein relate to the genotyping of a nucleic acid in a sample. In various embodiments, a sample is obtained from a human or non-human animal. For example, the sample may be a patient sample. A "patient sample" is any biological specimen from a patient. The term sample includes, but is not limited to, biological fluids such as blood, serum, plasma, urine, cerebrospinal fluid, tears, saliva, lymph, dialysis fluid, lavage fluid, semen, and/or other liquid samples, as well as cells and tissues of biological origin. Cells and tissues may include buccal cells, mouthwash collections, or skin cells, including hair follicles. The term also includes cells isolated from a human or cells derived therefrom, including cells in culture, cell supernatants, and cell lysates. It further includes organ or tissue culture-derived fluids, tissue biopsy samples, tumor biopsy samples, stool samples, and fluids extracted from physiological tissues, as well as cells dissociated from solid tissues, tissue sections, and cell lysates. It may also include post-mortem solid tissue samples, such as those from brain. The term sample also includes any other cellular or non-cellular specimen obtained from a human or non-human animal that comprises a nucleic acid of interest. In some embodiments, the sample contains less than about 80, 100, 150, 200, 500, 1,000, 1,500, 2,000, 2,500, 3,000, 4,000, or 5,000 ng of the nucleic acid of interest.

In some instances, the sample includes one or more nucleic acids of interest. The nucleic acid of interest can be genomic DNA. The genomic DNA or other nucleic acid of interest may be separated from other DNA and non-DNA components of the sample before being subjected to the methods of the invention. Many methods of DNA purification and separation are known in the art and may be used with the disclosed methods.

In some embodiments, the nucleic acid of interest in the sample may comprise the *FMR1* and/or *FMR2* genes or fragments thereof, or at least part of the 5' UTR of *FMR1* and/or *FMR2* (e.g., a portion that comprises the CGG repeats of the 5' UTR of *FMR1* or the CCG repeats in the 5' UTR of *FMR2*). In certain embodiments, the size of the nucleic acid may be about 50, 100, 200, 300, 500, or 700 bp, or 1, 1.5, 2, 2.5, 3, 4, 5, 7, or 10 kb, or any value in between. In some embodiments, the size of the nucleic acid may be between 50 bp and 10 kb, 100 bp and 10 kb, 200 bp and 10 kb, 300 bp and 10 kb, 500 bp and 10 kb, 700 bp and 10 kb, 1 kb and 10 kb, 1.5 bp and 10 kb, 2 bp and 10 kb, 3 bp and 10 kb, 50 bp and 7 kb, 50 bp and 5 kb, 50 bp and 4 kb, 50 bp and 3 kb, 50 bp and 2 kb, 50 bp and 1.5 kb, 100 bp and 7 kb, 200 bp and 5 kb, or 300 bp and 4 kb.

## III. Genotyping apparatus and machine-readable medium

In various embodiments, an apparatus is disclosed for use in the automated reconstruction of a genotype. In some embodiments, the apparatus comprises a processor communicatively coupled to a memory device. In some embodiments, machine-executable instructions are stored on the memory device that, when executed by the processor, cause the processor to conduct exhaustive enumeration analysis. In certain embodiments, the machine-executable instructions cause the processor to (a) accept the input of parameter information regarding the total length of a repeat or GC-rich region, the distance in the forward direction to any interruptions in the region, and the distance in the reverse direction to any interruptions in the region; (b) generate a set of potential genotypes comprising all possible arrangements of the interruptions in the repeat or GC-rich region; (c) evaluate the set of potential genotypes to produce a solution genotype that satisfies all the parameter information; and (d) store the solution genotype on the memory device or on any other memory device that is communicatively coupled to the processor. In certain embodiments, the apparatus further comprises a monitor communicatively coupled to the processor and memory device, wherein the machine-executable instructions stored on the memory device instruct the processor to display the solution genotype on the monitor. In some embodiments, the apparatus further comprising a printer communicatively coupled to the processor and memory device, wherein the machine-executable instructions stored on the memory device instruct the processor to print the solution genotype on the printer.

In various embodiments, the apparatus used to reconstruct a genotype is capable of accepting the input of parameter information regarding a nucleic acid (e.g., the length of any repeat or GC-rich regions and the distance from the start of the regions to each interruption in either the forward or reverse direction). In some embodiments, the apparatus is programmed to use the parameter information to reconstruct the genotype of the nucleic acid using exhaustive enumeration. The apparatus can be programmed to display and/or archive the reconstructed genotype. In some embodiments, the apparatus comprises a means for displaying and/or archiving the reconstructed genotype.

In various embodiments, an apparatus disclosed herein comprises a processor and memory device, wherein the memory device contains machine-readable instructions that instruct the processor to accept the input of parameter information regarding a nucleic acid and conduct exhaustive enumeration analysis, which can be represented by the formula $\pi|C_i|$, which indicates the product of the number of potential genotypes for the possible alleles, where $|C_i|$ is the number of candidate genotypes for allele i. As a result, this formula provides the number of possible genotypes that can be reconstructed from the parameter information (i.e., the set of potential genotypes that can be recreated using the length of the repeat or GC-rich region and the number of interruptions within the region in either the forward or reverse directions). In an embodiment, machine-readable instructions instruct the processor to generate a list of $C_i$ potential genotypes using the forward or reverse interruption information and then individually evaluate each potential genotype against the full set of parameter information to identify a solution genotype. In some embodiments, the apparatus further comprises a means to display the solution genotype (e.g., a monitor to display the genotype visually, a data storage medium to save the genotype in a digital format, and/or a connection for transmitting the solution genotype to a printer or other electronic storage or display device).

In some embodiments, the apparatus is a computer, wherein the computer comprises a processor and a memory device having computer code stored on it, wherein the computer code instructs the processor to accept the input of parameter information regarding a nucleic acid and then apply exhaustive enumeration to reconstruct a genotype for the nucleic acid. In some embodiments, the computer also comprises a monitor to display input information and/or the reconstructed genotype. In some embodiments, the reconstructed genotype is stored electronically on the computer and/or is capable of being printed for further diagnostic or therapeutic uses. In various embodiments, the computer comprises a device to allow for user interaction. For example, the computer may comprise a keyboard and/or pointing device (e.g., a mouse or a trackball) that allows a user (such as a patient, doctor, or other healthcare worker) to enter parameter information and/or to access and manipulate the reconstructed genotype.

In various embodiments, the instructions to conduct exhaustive enumeration may be stored on an apparatus in a machine-readable medium (e.g., machine-executable instructions, software, computer code, computer programs, etc.). For example, the machine-readable medium can comprise computer code stored in C++, JAVA, PERL, or any other suitable format for computer code. The machine-readable medium can provide instructions to the apparatus for conducting exhaustive enumeration using parameter information regarding a nucleic acid. In various embodiments, the instructions on the machine-readable medium can instruct an apparatus to (a) receive parameter information regarding the total length of a repeat or GC-rich region, the distance in the forward direction to any interruptions in the region, and the distance in the reverse direction to any interruptions in the region; (b) generate a set of potential genotypes comprising all possible arrangements of the interruptions in the repeat or GC-rich region; (c) evaluate the set of potential genotypes to produce a solution genotype that satisfies all the parameter information; and (d) store the solution genotype on a memory device. In some embodiments, the instructions on the machine-readable medium instruct the apparatus to display the solution genotype on a monitor. In some embodiments, the instructions on the machine-readable medium instruct the apparatus to print the solution genotype on a printer.

The instructions stored on a machine-readable medium can be any codes, symbols, or other signals that provide instructions, information, and/or data that can be used by an apparatus (e.g., by a processor in a computer). In some embodiments, the instructions stored on the machine-readable medium encode a program that instructs the apparatus to receive parameter information regarding a nucleic acid, conduct exhaustive enumeration analysis, and store or transmit a reconstructed genotype for the nucleic acid.

In some embodiments, the instructions stored on the machine-readable medium instruct the apparatus to execute an exhaustive enumeration analysis program, which can be represented by the formula $\pi|C_i|$, which indicates the product of the number of potential genotypes for the possible alleles, where $|C_i|$ is the number of candidate genotypes for allele i. As a result, this formula provides the number of possible genotypes that can be reconstructed from the parameter information (i.e., the set of potential genotypes that can be recreated using the length of the repeat or GC-rich region and the number of interruptions within the region in either the forward or reverse directions). In an embodiment, the program generates a list of $C_i$ potential genotypes using the forward or reverse interruption information and then individually evaluates each potential genotype against the full set of parameter information to identify a solution genotype. In some embodiments, the program includes instructions to display and/or archive the reconstructed solution genotype (e.g., to display the genotype on a monitor, to save the genotype to a data storage medium, and/or to transmit the solution genotype to a printer or other electronic storage or display device).

In some embodiments, the instructions stored on the machine-readable medium further encode a user interface that provides a graphical display on a monitor. In some embodiments, the interface allows a user to enter parameter information regarding a nucleic acid (e.g., by allowing the user to upload a data file or by allowing the user to enter information into display fields shown on the user interface). In some embodiments, the user interface provides the user with options for analyzing the parameter information, such as various methods for displaying and/or saving the input data and/or reconstructed genotypes (e.g., by displaying the data on the user's monitor, sending the data to a specified electronic device or electronic address, printing, and/or saving the data to a particular location).

In various embodiments, a reconstructed genotype can be stored as data in a storage medium physically connected to the apparatus (e.g., on an internal memory device such as a hard drive on a computer) and/or stored on a remote storage device that is communicatively connected to the apparatus (e.g., by a wired or wireless intranet or internet connection and the like). In some embodiments, the user interface provides the user with options for automatically storing the reconstructed genotype in a particular location, printing the genotype, and/or sending the genotype to a specified electronic device or electronic address (e.g., to the email address of the medical professional that requested the genotype reconstruction).

**IV. Methods of use**

In various embodiments, methods to detect a mutation or genotype comprising a GC-rich or repeat region, or to diagnose or treat a genetic disorder associated with a GC-rich or repeat region are provided, comprising (1) obtaining a sample from a patient; (2) isolating a nucleic acid from the sample that has one or more repeat or GC-rich regions, such as a region comprising CGG or CCG repeats; (3) collecting parameter information from the isolated nucleic acid; (4) entering the sequence information into an apparatus programmed to use exhaustive enumeration to reconstruct a genotype from the parameter information; and (5) using the reconstructed genotype to detect a mutation or genotype or to diagnose a genetic disorder associated with a mutation in a repeat or GC-rich region and/or predict the risk of a genetic disorder in patient or an offspring of the patient and/or make a suitable treatment decision based on the reconstructed genotype. For example, the method can comprise isolating an *FMR1* or *FMR2* nucleic acid or fragments thereof from a patient sample, determining parameter information for the *FMR1* or *FMR2* nucleic acid, applying exhaustive enumeration to the parameter information to generate a reconstructed genotype for the repeat region in *FMR1* or *FMR2,* and using the reconstructed genotype to detect a mutation or genotype associated with an *FMR1* or *FMR2* genetic disorder, or to diagnose and/or predict the risk of and/or make treatment decisions regarding an associated disorder.

Numerous genes and genomic regions comprise repeat or GC-rich regions and are associated with genetic disorders, making them potential diagnostic and therapeutic targets. Accordingly, in various embodiments the exhaustive enumeration methods disclosed herein can be used to reconstruct genotypes for these genetic loci and can be used to diagnose, prognose, and/or guide treatment decisions for the associated genetic disorders. In some embodiments, the exhaustive enumeration methods disclosed herein can be used to reconstruct a genotype for the repeat region(s) of the *FMR1* or *FMR2* genes. In some embodiment, these reconstructed genotypes can assist in the diagnosis of FXS, FRAXE, FXTAS, FXPOI, and dopamine-responsive Parkinsonism, which are associated with the length of CGG repeat regions in the 5' UTR of *FMR1* and CCG repeat regions in the 5' UTR of *FMR2.* For example, a reconstructed *FMR1* genotype having greater than about 45 CGG repeats, and particularly a genotype having greater than about 200 CGG repeats, in the 5' UTR can be used to diagnose FXS and associated disorders.

In further embodiments, the exhaustive enumeration methods may be used to detect genotypes associated with other disorders of repeat or GC-rich regions, such as spinocerebellar ataxia type 1, spinocerebellar ataxia type 2, spinocerebellar ataxia type 3, spinocerebellar ataxia type 6, spinocerebellar ataxia type 7, spinocerebellar ataxia type 8, Friedrich's ataxia, progressive myoclomus epilepsy, amyotrophic lateral sclerosis, myotonic dystrophy, Huntington's disease, spinobulbar muscular atrophy, Dentatorubropallidoluysian atrophy, and/or spinocerebellar ataxia. Genetic loci associated with these conditions are known in the art and include, without limitation, *SCA1, SCA2, SCA3, CACNA1A, SCA7, SCA8, X25, CSTB, C90RF72, DMPK, ZNF9, HTT, AR, ATN1, ATXN1-3, ATXN7, ATXN10, CACNA1A, SCA8, PPP2R2B,* and *TBP. See, e.g.,* Nat Genet. 1996 May;13(1):105-8; Nat Genet. 1996 May;13(1):109-13. Hyperexpansion and/or hypermethylation of the GC-rich and/or repeat regions at these loci are associated with the diseases. Table 6 shows examples of genetic loci that can be used with the exhaustive enumeration methods disclosed herein, and the relationship between GC-rich and/or repeat regions in those loci and disease genotypes or phenotypes.

**Table 6**

| Disease | Gene | Repeat number | | Repeat position | Repeat variant |
|---|---|---|---|---|---|
| | | **Normal** | **Mutant** | | |
| Fragile X syndrome | *FMR1* | (CGG)<45 | (CGG)>200 | 5'-UTR | AGG |
| Fragile X (FRAXE) mental retardation | *FMR2* | (CCG)<35 | (CCG)>200 | 5'-UTR | CTG |

(continued)

| Disease | Gene | Repeat number | | Repeat position | Repeat variant |
|---|---|---|---|---|---|
| | | Normal | Mutant | | |
| Myotonic dystrophy | *DMPK* | (CTG)<35 | (CTG)>50 | 3'-UTR | CCG, CTC |
| Spinocerebellar ataxia type 8 | *SCA8* | (CTG)<40 | (CTG)>110 | Antisense RNA | CCG CTA, CTC, CCA or CTT |
| Friedrich's ataxia | *X25* | (GAA)<35 | (GAA)>100 | Intron 1 | GGA, GAG |
| Spinobulbar muscular atrophy | *AR* | (CAG)<30 | (CAG)>40 | Coding | |
| Huntington disease | *IT15* | (CAG)<40 | CAG)>40 | Coding | |
| Dentatorubral pallidoluysian atrophy | *DRPLA* | (CAG)<35 | (CAG)>50 | Coding | |
| Spinocerebelllar ataxia type 1 | *SCA1* | (CAG)<40 | (CAG)>40 | Coding | CAT |
| Spinocerebelllar ataxia _type 2 | *SCA2* | ataxia (CAG) <30 | (CAG)>35 | Coding | CAA |
| Spinocerebelllar ataxia type 3 | *SCA3* | (CAG)<40 | (CAG)>40 | Coding | |
| Spinocerebellar ataxia type 6 | *CACNA1A* | (CAG)<20 | (CAG)>20 | Coding | |
| Spinocerebelllar ataxia tvpe 7 | *SCA7* | (CAG)<40 | (CAG)>40 | Coding | normal allele has no interruption |
| Progressive myoclomus epilepsy type | *CSTB* | $(C_4GC_4GCG$ (SEQ ID NO: 66))<3 | $(C_4GC_4GCG$ (SEQ ID NO: 66)>50 | Promoter | |

For example, exhaustive enumeration can be used to detect genotypes associated with disorders of *SCA1* or *SCA2*, such as Spinocerebelllar ataxia types 1 and 2, which are associated with expansion of their CAG repeat regions. For example, parameter information can be provided regarding the total length of one or more CAG repeats in the *SCA1* or *SCA2* genes, as well as the distance in the forward and reverse directions to the CAT or CAA interruptions in the CAG repeats. Exhaustive enumeration, using the total length of the one or more CAG repeats and either the distance in the forward or reverse direction to any interruptions, can be applied to generate a set of potential genotypes for the *SCA1* or *SCA2* gene. The potential genotypes can be evaluated to determine a solution genotype that satisfies all the parameter information. The identified solution genotype can be used to detect a mutation or a genotype, or to diagnose or assist in diagnosing, an *SCA1* or SCA2 related mutation, genotype, or disorder.

**Examples**

The following examples serve to illustrate, and in no way limit, the present disclosure.

**Example 1: Exhaustive enumeration of a CGG locus in *FMR1***

Standard PCR and capillary electrophoresis (CE) analysis of the *FMR1* gene may provide parameter information characterizing a CGG repeat locus, such as: (1) the overall length of the CGG repeat locus, (2) Anchored A ("Anch A") mapping, and (3) Anchored T ("Anch T") mapping. The overall length data reveals the total length of the CGG repeat region (including any AGG interruptions). The Anch T data indicates the locations of AGGs within the CGG locus in the forward direction, while the Anch A data indicates the locations of the AGGs in the reverse direction.

Table 1 shown below is an example of an *FMR1* CGG locus having a total repeat length of 23 trinucleotides, with two AGG interruptions. Table 1 shows the position counts in the forward and reverse directions from the start and end of the CGG repeat locus to each of the AGG interruptions.

**Table 1**

| | | | | | | | | | | | |
|---|---|---|---|---|---|---|---|---|---|---|---|
| 1 | 2 | 3 | 4 | 5 | 6 | 7 | 8 | 9 | 10 | 11 | 12 |
| CGG | CGG | CGG | CGG | AGG | CGG | CGG | CGG | CGG | CGG | CGG | CGG |
| 23 | 22 | 21 | 20 | 19 | 18 | 17 | 16 | 15 | 14 | 13 | 12 |
| | | | | | | | | | | | |
| 13 | 14 | 15 | 16 | 17 | 18 | 19 | 20 | 21 | 22 | 23 | |
| CGG | CGG | CGG | CGG | CGG | AGG | CGG | CGG | CGG | CGG | CGG | |
| 11 | 10 | 9 | 8 | 7 | 6 | 5 | 4 | 3 | 2 | 1 | |
| Table 1 discloses SEQ ID NO: 47. | | | | | | | | | | | |

The CE report for this example would indicate an overall CGG repeat length of {23}, corresponding to the length of the CGG repeat locus. The Anch T assay would report {5,18}, corresponding to the positions of the AGG interruptions within the CGG repeat region, counting in the forward direction. The Anch A assay would report {6,19}, corresponding to the positions of the AGG interruptions when counting in the reverse direction. Given this CE report, the configuration of the CGG repeat locus can be reconstructed relatively easily by using the total CGG length and either the Anch A or Anch T data. The genotype can be written using compact notation as $(CGG)_4(AGG)(CGG)_{12}(AGG)(CGG)_5$ (SEQ ID NO: 47), where the subscripts represent the number of continuous CGG repeats before arriving at an AGG interruption.

While the genotype of an *FMR1* gene having a single CGG locus can be manually reconstructed fairly easily, as shown above, the reconstruction task becomes more complex when more than one CGG repeat locus is involved. For instance, as discussed in Example 3, the investigators required over 150 hours to manually reconstruct approximately 1000 FMR1 samples, indicating a rate of one sample per 10 minutes to manually reconstruct a single *FMR1* genotype in that experiment. Additional time was required to conduct quality assurance and error checking of the manually reconstructed genotypes. Even with quality control, a risk of user error is possible during manual reconstruction, especially if the process is performed by less well-trained lab technicians who may be required to screen through numerous patient samples. In contrast, the exhaustive enumeration software was able to reconstruct genotypes for nearly 1000 samples in that experiment in less than 10 minutes, translating into an approximately thousand-fold time savings.

**Example 2: Exhaustive enumeration of two CGG loci in *FMR1***

It is possible to have more than one CGG repeat locus in an *FMR1* gene, and this can greatly increase the number of potential genotypes that must be evaluated. For example, an *FMR1* gene could have the following two CGG loci:

Locus 1: $(CGG)_{19}(AGG)(CGG)_9$ (SEQ ID NO: 48)

Locus 2 : $(CGG)_{10}(AGG)(CGG)_{41}$ (SEQ ID NO: 49)

The complete CE report for this sample is shown in Table 2.

**Table 2**

| | |
|---|---|
| CGG_Locus_Length | {29,52} |
| Anch_T | {11, 20} |
| Anch_A | {10, 42} |

Merely using the information shown in Table 2, it is not a simple task to reconstruct the *FMR1* genotype. In particular, it is not clear which components of the Anch T report (or the Anch A report) correspond to interruptions in either of the two CGG repeat loci. Instead, exhaustive enumeration software is used to identify the correct genotype.

First, the software enumerates all possible genotypes for each of the two loci using the overall CGG repeat length and Anch T information. Next, the solutions are evaluated for self consistency and report compatibility. Self consistency asks whether the potential solution genotype has one or more consecutive AGG sequence and rejects any potential sequence having consecutive AGGs. Report compatibility asks whether the potential solution genotype is fully consistent with all data in the CE report. In other words, report compatibility asks whether a potential solution is consistent with the CGG locus length, Anch T, and Anch A data. The genotype that satisfies all three requirements is selected as the solution genotype.

Using the overall CGG repeat length and Anch T data shown in Table 2, the exhaustive enumeration program would generate sets of potential sequences for CGG loci 1 and 2, as shown in Tables 3 and 4.

**Table 3: Potential sequences for CGG locus 1**

| | |
|---|---|
| $(CGG)_{29}$ (SEQ ID NO: 50) | Corresponding to a genotype lacking interruptions |
| $(CGG)_{10}(AGG)(CGG)_{18}$ (SEQ ID NO: 51) | Corresponding to a genotype having one interruption at position {11} |
| $(CGG)_{19}(AGG)(CGG)_{9}$ (SEQ ID NO: 48) | Corresponding to a genotype having one interruption at position {20} |
| $(CGG)_{10}(AGG)(CGG)_{8}(AGG)(CGG)_{9}$ (SEQ ID NO: 52) | Corresponding to a genotype having two interruption at positions {11, 20} |

**Table 4: Potential sequences for CGG locus 2**

| | |
|---|---|
| $(CGG)_{52}$ (SEQ ID NO: 53) | Corresponding to a genotype lacking interruptions |
| $(CGG)_{10}(AGG)(CGG)_{41}$ (SEQ ID NO: 49) | Corresponding to a genotype having one interruption at position {11} |
| $(CGG)_{19}(AGG)(CGG)_{32}$ (SEQ ID NO: 54) | Corresponding to a genotype having one interruption at position {20} |
| $(CGG)_{10}(AGG)(CGG)_{8}(AGG)(CGG)_{32}$ (SEQ ID NO: 55) | Corresponding to a genotype having two interruption at positions {11, 20} |

Combining Tables 3 and 4 generates a set of 16 potential genotypes derived from the CE data for the *FMR1* gene having 2 CGG repeat loci, as shown in Table 5.

**Table 5**

| |
|---|
| $(CGG)_{29}$ (SEQ ID NO: 50) / $(CGG)_{52}$ (SEQ ID NO: 53) |
| $(CGG)_{29}$ (SEQ ID NO: 50) / $(CGG)_{10}(AGG)(CGG)_{41}$ (SEQ ID NO: 49) |
| $(CGG)_{29}$ (SEQ ID NO: 50) / $(CGG)_{19}(AGG)(CGG)_{32}$ (SEQ ID NO: 54) |
| $(CGG)_{29}$ (SEQ ID NO: 50) / $(CGG)_{10}(AGG)(CGG)_{8}(AGG)(CGG)_{32}$ (SEQ ID NO: 55) |
| $(CGG)_{10}(AGG)(CGG)_{18}$ (SEQ ID NO: 51) / $(CGG)_{52}$ (SEQ ID NO: 53) |
| $(CGG)_{10}(AGG)(CGG)_{18}$ (SEQ ID NO: 51) / $(CGG)_{10}(AGG)(CGG)_{41}$ (SEQ ID NO: 49) |
| $(CGG)_{10}(AGG)(CGG)_{18}$ (SEQ ID NO: 51) / $(CGG)_{19}(AGG)(CGG)_{32}$ (SEQ ID NO: 54) |
| $(CGG)_{10}(AGG)(CGG)_{18}$ (SEQ ID NO: 51) / $(CGG)_{10}(AGG)(CGG)_{8}(AGG)(CGG)_{32}$ (SEQ ID NO: 55) |
| $(CGG)_{19}(AGG)(CGG)_{9}$ (SEQ ID NO: 48) / $(CGG)_{52}$ (SEQ ID NO: 53) |
| $(CGG)_{19}(AGG)(CGG)_{9}$ (SEQ ID NO: 48) / $(CGG)_{10}(AGG)(CGG)_{41}$ (SEQ ID NO: 49) |
| $(CGG)_{19}(AGG)(CGG)_{9}$ (SEQ ID NO: 48) / $(CGG)_{19}(AGG)(CGG)_{32}$ (SEQ ID NO: 54) |
| $(CGG)_{19}(AGG)(CGG)_{9}$ (SEQ ID NO: 48) / $(CGG)_{10}(AGG)(CGG)_{8}(AGG)(CGG)_{32}$ (SEQ ID NO: 55) |
| $(CGG)_{10}(AGG)(CGG)_{8}(AGG)(CGG)_{9}$ (SEQ ID NO: 52) / $(CGG)_{52}$ (SEQ ID NO: 53) |
| $(CGG)_{10}(AGG)(CGG)_{8}(AGG)(CGG)_{9}$ (SEQ ID NO: 52) / $(CGG)_{10}(AGGCGG)_{41}$ (SEQ ID NO: 49) |
| $(CGG)_{10}(AGG)(CGG)_{8}(AGG)(CGG)_{9}$ (SEQ ID NO: 52) / $(CGG)_{19}(AGG)(CGG)_{32}$ (SEQ ID NO: 54) |
| $(CGG)_{10}(AGG)(CGG)_{8}(AGG)(CGG)_{9}$ (SEQ ID NO: 52) / $(CGG)_{10}(AGG)(CGG)_{8}(AGG)(CGG)_{32}$ (SEQ ID NO: 55) |

Each of the genotypes in Table 5 is evaluated for self consistency and report compatibility. For example, the first potential genotype ($(CGG)_{29}$ (SEQ ID NO: 50) / $(CGG)_{52}$ (SEQ ID NO: 53)) is self-consistent since the genotype does not contain any contiguous AGG sequences. However, the genotype generates an Anch T of {null} and an Anch A of {null} because there are no AGG interruptions in either loci, which is not consistent with the Anch T of {11, 20} and Anch A of {10, 42} shown in the CE report. Thus, this potential solution is rejected as report incompatible. Similarly, the next potential solution, ($(CGG)_{29}$ (SEQ ID NO: 50) / $(CGG)_{10}$(AGG)$(CGG)_{41}$ (SEQ ID NO: 49)) is also self consistent but not report compatible, as it generates an Anch T of {11} and an Anch A of {42}. Thus, the software would also reject this potential solution as report incompatible. The software continues to examines each of the 16 potential genotypes and rejects all of them except for $(CGG)_{19}$(AGG)$(CGG)_9$ (SEQ ID NO: 48) / $(CGG)_{10}$(AGG)$(CGG)_{41}$ (SEQ ID NO: 49), which is self consistent and report compatible (it generates an Anch T of {11, 20} and an Anch A of {10, 42}, which correspond to the Anch A and Anch T data in the CE report). This sequence is identified as the solution genotype.

**Example 3: Application of Exhaustive Enumeration in 887 FMR1 samples**

To validate the accuracy of the exhaustive enumeration software, 887 samples (from mothers, fathers, and offspring in families linked to FMR1 disorders) were independently analyzed for genotype using exhaustive enumeration software and compared to the results of manual interpretation. Nolin et al., Am. J. Med. Genet. Part A 9999: 1-8 (2013). The software produced a significant time savings, requiring less than 10 minutes to reconstruct genotypes for all 887 samples, whereas the investigators manually reconstructed the same samples at a much slower rate. On average, only a single FMR1 genotype was reconstructed in a similar (10') time period. Additional time was required to conduct quality assurance and error checking of the manually reconstructed genotypes.

The samples used in the study were obtained as purified gDNA from the blood of donors and represented alleles in the normal, intermediate/premutation range (i.e., lengths of 35-69 CGG repeats), and full mutations.

Parameter data was generated using three PCR assays and capillary electropherogram traces to resolve the AGG status of each allele. The assays included a CGG repeat primed PCR (Tassone et al., J. Mol. Diagn. 10: 43-49 (2008); Chen et al., J. Mol. Diagn. 12: 589-600 (2010)) and two additional PCR assays derived from a previously validated long-read PCR technique (Filipovic-Sadic et al., Clin. Chem. 56: 399-408 (2010)) that resolved ambiguities in the repeat primed assay. The DNA samples were diluted to 20 ng/ml and a total of 120 ng of DNA was used in each PCR reaction. Electropherogram signals were generated for the PCR products using a 3500XL Genetic Analyzer (Life Technologies, Carlsbad, CA). In general, samples were batched in groups of 94 samples and two controls per microtiter plate. Using three thermal cyclers and one 3500XL, the turnaround time for data acquisition was 24-36 hours per sample batch. A total of 99.0% of the samples (878/887) were correctly identified using the software (when compared to the manual reconstructions), and 96.8% (859/887) of the samples produced a unique, correct solution.

An example of the parameter data generated for a sample where the genotype was correctly identified using the exhaustive enumeration software is shown in Fig. 1. The CGG Assay indicated a single CGG repeat region having a total length of 42 repeats, while the Anch_A assay indicated an AGG interruption at position 42 and the Anch_T assay indicated an AGG interruption at position 10. The genotype produced by the software was $(CGG)_9$AGG$(CGG)_{32}$ (SEQ ID NO: 56) and this matched the sequence decoded manually.

In 1% of the tested samples, the software did not produce a single solution genotype. In those samples, further evaluation revealed an AGG interruption occurring after less than 5 CGG repeats, reflecting a detection limitation of the PCR methods used to analyze the sample in this experiment. The forward primer used in the Anch_A assay contained the sequence $(CGG)_5$A (SEQ ID NO: 42). For samples containing an AGG interruption after less than 5 CGG repeats, the primer did not anneal and a unique PCR product was not produced for that stretch of the CGG region (the Anch_A assay did not report an interruption, but rather a longer stretch of CGG repeats with a length depending on the location of the next AGG interruption). Similarly, the reverse primer used in the Anch_T assay contained the sequence (CCG)4CCT (SEQ ID NO: 57) and therefore required a stretch of at least 4 CGG repeats between AGG interruptions in order to anneal.

For instance, Fig. 2 shows the PCR data generated for a sample comprising the sequence $(CGG)_6$AGG$(CGG)_2$AGG$(CGG)_{41}$ (SEQ ID NO: 58). The CGG repeat assay correctly identified the total length of the CGG region at 51 repeats. The box in the CGG assay indicates an 8 peak dip in peak height, larger than the 5 peak dip normally seen in the CGG assay when a single AGG interruption is present. The normal 5 peak dip is caused by insufficient binding of the primer used in the CGG assay (containing a $(CGG)_5$ (SEQ ID NO: 59) repeat sequence) to the AGG-containing sequence at each possible target position along the 5 repeat stretch. In the Anch_A assay, the forward primer did not bind in the $(CGG)_2$ region, and hence the peak at position 42 was not identified, only a single peak at position 45. Likewise, the reverse primer in the Anch_T assay did not bind in the $(CGG)_2$ region, and thus did not show a peak at position 7, only one at position 10. The exhaustive enumeration software therefore could not generate a correct solution genotype. To address this limitation, alternative primers may be designed that do not require a similar length CGG repeat sequence but are still specific for the region near an AGG interruption.

In a small percentage of cases (less than 4%), the exhaustive enumeration software generated more than one solution genotype. An example of the PCR data from a sample where the software produced two genotypes is shown in Fig. 3. The CGG assay data shows two alleles, one having 29 CGG repeats and one having 36 CGG repeats. The two solution genotypes reported by the software were

First solution genotype:

Allele 1 = $(CGG)_9AGG(CGG)_9AGG(CGG)_9$ (SEQ ID NO: 60)

Allele 2 = $(CGG)_9AGG(CGG)_9AGG(CGG)_6AGG(CGG)_9$ (SEQ ID NO: 61)

Second solution genotype:

Allele 1 = $(CGG)_9AGG(CGG)_{19}$ (SEQ ID NO: 62)

Allele 2 = $(CGG)_9AGG(CGG)_9AGG(CGG)_6AGG(CGG)_9$ (SEQ ID NO: 61)

Manual genotype reconstruction confirmed that the first solution genotype was correct. The correct genotype was also be identified by comparing the peak heights in the electropherogram data of the CGG Assay against the genotype solutions identified by the exhaustive enumeration software (see Example 4 below for an explanation of the use of peak height and peak pattern in genotype reconstruction).

While the automated software reported more than one genotype in a small percentage of cases, and further manual analysis identified a single solution, the manual workload was still significantly reduced. Rather than manually evaluating every possible genotype suggested by the PCR data, the investigators only needed to manually evaluate the more limited set of solution genotypes generated by the software where multiple solution genotypes were initially identified. Moreover, additional automation steps may be added to the software to enable automated evaluation of the CGG peak height and peak pattern to identify the single correct solution genotype, as discussed in Example 4 below. Thus, even when multiple genotypes are reported by the software, the amount of manual operator time required to identify the single correct genotype, and the risk of operator error, is reduced.

**Example 4: Resolution of Samples with More Than One Solution Genotype**

In experimental instances where the exhaustive enumeration software produced more than one solution, the inventors resolved the correct genotype by evaluating the peak height pattern produced by the CGG assay. The raw CGG assay electropherogram data provides a series of signal peaks of decreasing intensity as PCR product length increases, with a drop to background signal levels beyond the length of the overall allele. The peak height pattern can be modeled using a polynomial function such as a cubic fit. When the allele contains an AGG interruption, this is indicated by a dip in peak height for 5 peaks in the CGG assay, using a primer that is complementary to a region containing 5 CGG repeats. The 5 peak dip is caused by insufficient binding of the primer used in the CGG assay (complementary to a $(CGG)_5$ (SEQ ID NO: 59) repeat sequence) to the AGG-containing sequence at each possible target position along the 5 repeat stretch. By evaluating this pattern of peak heights in the CGG length assay, the investigators were able to manually select the correct genotype for those samples from the 887 tested in Example 3 where the exhaustive enumeration software provided more than one solution sequence.

This manual evaluation may also be automated and incorporated into the exhaustive enumeration software. For instance, for each of the K possible genotypes generated by exhaustive enumeration, the software can be programmed to generate a polynomial curve that best fits the CGG assay data and comports with the particular genotype. Error (i.e., the gap between the polynomial curve and the actual CGG assay data) can be computed for each curve, and the software can then select the curve that minimizes error, identifying the correct solution genotype.

The error (E) in a curve can be computed using the formula $E=[e_1,...,e_k,..., e_K]$, wherein $e_k$ represents the error between the polynomial curve and the actual CGG peak height at a particular position. Minimized $e_k$ for a genotype can be represented by the formula $e_k = \min_P d(X_k * P - O)$ where P is the polynomial curve parameter vector, O the observed peak data, and $X_k$ represents the solution structure matrix for each solution genotype identified by the exhaustive enumeration software. The solution structure matrix is the concatenation of the individual allele structure matrices: $Xa=[Xa_1|Xa_2| ...]$. The allele structure matrix X is a columnwise concatenation of positional indexes of peaks and its higher powers (up to the desired degree) for each of the alleles , i.e. $Xa_1 = [I(a)|a|a^2|a^3| ...]$. For each allele in the solution a vector a is defined as [5,6,... allele length] for alleles with no interspersions; in case of interspersions the corresponding sequence of 5 peaks is set to zero. The distance parameter d can be a simple Lp norm or a robust regression objective function.

An example applying this automated method is described below. For example, given the following PCR data, the exhaustive enumeration software will produce two solution genotypes:

CGG length assay ={30,40}

Anch_T_assay={11,12,21}

Anch_A_assay={10,20,29}

The two solution genotypes are:

Genotype 1 :

Allele 1 : $(CGG)_{10}(AGG)(CGG)_{9}(AGG)(CGG)_{9}$ (SEQ ID NO: 63)

Allele 2 : $(CGG)_{11}(AGG)(CGG)_{28}$ (SEQ ID NO: 64)

Genotype 2 :

Allele 1 : $(CGG)_{10}(AGG)(CGG)_{9}(AGG)(CGG)_{9}$ (SEQ ID NO: 63)

Allele 2 : $(CGG)_{11}(AGG)(CGG)_{8}(AGG)(CGG)_{19}$ (SEQ ID NO: 65)

To determine the correct solution, the peak height data in Table 7, taken from the raw CGG assay electropherogram data, is used to provide a best fit curve for each of the two genotypes identified by the software:

**Table 7**

| Location | Observed Peak Height |
|---|---|
| 5 | 6477 |
| 6 | 5922 |
| 7 | 5756 |
| 8 | 5503 |
| 9 | 5295 |
| 10 | 3193 |
| 11 | 2209 |
| 12 | 2052 |
| 13 | 2124 |
| 14 | 2252 |
| 15 | 4601 |
| 16 | 4596 |
| 17 | 4327 |
| 18 | 4301 |
| 19 | 3937 |
| 20 | 2441 |
| 21 | 1795 |
| 22 | 1745 |
| 23 | 1723 |
| 24 | 1974 |

(continued)

| Location | Observed Peak Height |
|---|---|
| 25 | 3477 |
| 26 | 3639 |
| 27 | 3319 |
| 28 | 2890 |
| 29 | 1971 |
| 30 | 1278 |
| 31 | 255 |
| 32 | 153 |
| 33 | 547 |
| 34 | 1676 |
| 35 | 1709 |
| 36 | 1716 |
| 37 | 1609 |
| 38 | 1547 |
| 39 | 1360 |
| 40 | 997 |

For each of the 2 genotypes, the software generates Xa matrices. The parameter values (P) for each of the two solutions that minimize the robust mean squared error $e_k$ between the observed and best fit in each matrix are P1=[2070.52, 116.22, -7.63, 0.10, 5545.94, -432.51, 16.75, -0.22]; and P2=[-1361.32, 550.63, -20.60, 0.18, 9362.48, -967.56, 37.00, -0.45]. The mean square errors themselves are $e_1$=57161.39 and $e_2$=568727.9. The best fit curve for each of the two genotypes is shown in Fig. 4. Using this result, the software identifies the first genotype as the one having lower mean squared error and is therefore the correct genotype.

The preceding examples are intended to illustrate and in no way limit the present disclosure. Other embodiments of the disclosed devices and methods will be apparent to those skilled in the art from consideration of the specification and practice of the devices and methods disclosed herein.

## Claims

**1.** A method of automated reconstruction of a genotype, comprising

a. providing a nucleic acid isolated from a patient sample, wherein the nucleic acid has at least one GC-rich nucleotide repeat region comprising one or more interruptions;
b. determining parameter information for the nucleic acid, wherein determining parameter information comprises measuring the total length of the at least one GC-rich nucleotide repeat region, the distance from the start of the at least one GC-rich nucleotide repeat region to the one or more interruptions in the region, and the distance from the end of the at least one GC-rich nucleotide repeat region to the one or more interruptions in the region; and
c. using an apparatus to conduct automated exhaustive enumeration on the parameter information to generate a reconstructed genotype, wherein exhaustive enumeration comprises

i) using the total length of the at least one GC-rich nucleotide repeat region and either the distance from the start of the at least one GC-rich nucleotide repeat region to the one or more interruptions in the region or the distance from the end of the at least one GC-rich nucleotide repeat region to the one or more interruptions in the region to generate a set of potential genotypes comprising all possible arrangements of the one or more interruptions in the region; and
ii) evaluating the set of potential genotypes to determine a solution genotype that satisfies all the parameter information.

**2.** The method of claim 1, wherein the parameter information is determined by polymerase chain reaction.

**3.** The method of claim 1, wherein the apparatus comprises a processor and a memory device communicatively coupled to the processor, wherein the memory device has stored therein machine-executable instructions that, when executed by the processor, cause the processor to receive parameter information and to conduct an exhaustive enumeration analysis.

**4.** The method of claim 1, further comprising confirming the reconstructed genotype by manually comparing the genotype to the parameter information, by performing a restriction digest, or by sequencing the nucleic acid having at least one GC-rich nucleotide repeat region.

**5.** The method of claim 1, wherein the genotype being reconstructed is:

a. the *FMR1* gene or a fragment thereof, wherein the sample comprises an *FMR1* nucleic acid or a fragment thereof, and wherein the parameter information comprises determining the total length of at least one CGG repeat region in the *FMR1* nucleic acid or fragment, the distance from the start of the at least one CGG repeat region to one or more AGG interruptions in the region, and the distance from the end of the at least one CGG repeat region to one or more AGG interruptions in the region, or
b. the *FMR2* gene or a fragment thereof, wherein the sample comprises an *FMR2* nucleic acid or a fragment thereof, and wherein the parameter information comprises determining the total length of at least one CCG repeat region in the *FMR2* nucleic acid or fragment, the distance from the start of the at least one CCG repeat region to one or more AGG interruptions in the region, and the distance from the end of the at least one CCG repeat region to one or more AGG interruptions in the region.

**6.** The method of claim 5, further comprising using the reconstructed genotype to detect a genotype associated with an *FMR1* or *FMR2* disorder or phenotype in a patient, or to detect a risk of an *FMR1* or *FMR2* disorder or phenotype in offspring of the patient.

**7.** The method of claim 6, wherein the *FMR1* or *FMR2* disorder is Fragile X Syndrome (FXS), Fragile X syndrome E (FRAXE), Fragile X-associated tremor/ataxia syndrome (FXTAS), fragile X-related primary ovarian insufficiency (FXPOI), or dopamine-responsive Parkinsonism.

**8.** An apparatus for automated reconstruction of a genotype, comprising

a. a processor; and
b. a memory device communicatively coupled to the processor, the memory device having stored therein machine-executable instructions that, when executed by the processor, cause the processor to

i. receive parameter information for a nucleic acid, where the nucleic acid has at least one GC-rich nucleotide repeat region comprising one or more interruptions; and wherein the parameter information comprises the total length of the at least one GC-rich nucleotide repeat region, the distance from the start of the at least one GC-rich nucleotide repeat region to the one or more interruptions in the region, and the distance from the end of the at least one GC-rich nucleotide repeat region to the one or more interruptions in the region;
ii. generate a set of potential genotypes comprising all possible arrangements of the one or more interruptions in the region by conducting automated exhaustive enumeration on the parameter information;

iii. evaluate the set of potential genotypes to produce a solution genotype that satisfies all the parameter information; and
iv. store the solution genotype on the memory device.

**9.** The apparatus of claim 8, further comprising a monitor communicatively coupled to the processor and memory device, wherein the machine-executable instructions stored on the memory device instruct the processor to display the solution genotype on the monitor.

**10.** The apparatus of claim 8, further comprising a printer communicatively coupled to the processor and memory device, wherein the machine-executable instructions stored on the memory device instruct the processor to print the solution genotype on the printer.

**11.** A machine-readable medium comprising machine-executable instructions that, when executed by a processor, causes the processor to

a. receive parameter information for a nucleic acid, wherein the nucleic acid has at least one GC-rich nucleotide repeat region comprising one or more interruptions; and wherein the parameter information comprises the total length of the at least one GC-rich nucleotide repeat region, the distance from the start of the at least one GC-rich nucleotide repeat region to the one or more interruptions in the region, and the distance from the end of the at least one GC-rich nucleotide repeat region to the one or more interruptions in the region;
b. generate a set of potential genotypes comprising all possible arrangements of the one or more interruptions in the region by conducting automated exhaustive enumeration on the parameter information
c. evaluate the set of potential genotypes to produce a solution genotype that satisfies all the parameter information; and
d. store the solution genotype on a memory device.

**12.** The machine-readable medium of claim 11, wherein the machine-executable instructions instruct the processor to display the solution genotype on a monitor.

**13.** The machine-readable medium of claim 11, wherein the machine-executable instructions instruct the processor to print the solution genotype on a printer.

**14.** A kit comprising the apparatus of any one of claims 8-10, or the machine readable medium of any one of claims 11-13, and instructions for using the apparatus or machine readable medium to reconstruct a genotype.

**15.** The kit of claim 14, wherein the kit is used to detect a genotype associated with an *FMR1* or *FMR2* disorder or phenotype in a patient, or to detect a risk of an *FMR1* or *FMR2* disorder or phenotype in offspring of the patient.

**Patentansprüche**

**1.** Verfahren zur automatisierten Rekonstruktion eines Genotyps, umfassend

a. Bereitstellen einer von einer Patientenprobe isolierten Nukleinsäure, wobei die Nukleinsäure zumindest einen GC-reichen Nukleotid-Repeat-Bereich aufweist, der eine oder mehrere Unterbrechungen umfasst;
b. Bestimmen der Parameterinformation für die Nukleinsäure, wobei das Bestimmen der Parameterinformation das Messen der Gesamtlänge des zumindest einen GC-reichen Nukleotid-Repeat-Bereichs, der Entfernung vom Beginn des zumindest einen GC-reichen Nukleotid-Repeat-Bereichs zu einer oder mehreren Unterbrechungen in dem Bereich und der Entfernung vom Ende des zumindest einen GC-reichen Nukleotid-Repeat-Bereichs zu einer oder mehreren Unterbrechungen in dem Bereich umfasst; und
c. Verwenden eines Apparats, um eine automatisierte umfassende Aufzählung der Parameterinformation durchzuführen, um einen rekonstruierten Genotyp zu erzeugen, wobei die umfassende Aufzählung Folgendes umfasst

i) Verwenden der Gesamtlänge des zumindest einen GC-reichen Nukleotid-Repeat-Bereichs und entweder der Entfernung vom Beginn des zumindest einen GC-reichen Nukleotid-Repeat-Bereichs zu einer oder mehreren Unterbrechungen in dem Bereich oder der Entfernung vom Ende des zumindest einen GC-reichen Nukleotid-Repeat-Bereichs zu einer oder mehreren Unterbrechungen in dem Bereich, um einen Satz von potenziellen Genotypen zu erzeugen, die alle möglichen Anordnungen von der einen oder mehreren Unterbrechungen in dem Bereich umfassen; und
ii) Auswerten des Satzes von potenziellen Genotypen, um einen Lösungsgenotyp zu bestimmen, der alle Parameterinformationen erfüllt.

**2.** Verfahren nach Anspruch 1, wobei die Parameterinformation durch Polymerasekettenreaktion bestimmt wird.

**3.** Verfahren nach Anspruch 1, wobei der Apparat einen Prozessor und eine Speichervorrichtung umfasst, die kommunikativ an den Prozessor gekoppelt ist, wobei die Speichervorrichtung darin maschinenausführbare Anweisungen gespeichert hat, die, wenn sie von dem Prozessor ausgeführt werden, den Prozessor veranlassen, Parameterinformationen zu empfangen und eine umfassende Aufzählungsanalyse durchzuführen.

**4.** Verfahren nach Anspruch 1, ferner umfassend das Bestätigen des rekonstruierten Genotyps durch manuelles Vergleichen des Genotyps mit der Parameterinformation, durch Durchführen eines Restriktionsverdaus oder durch Sequenzieren der Nukleinsäure, die zumindest einen GC-reichen Nukleotid-Repeat-Bereich aufweist.

**5.** Verfahren nach Anspruch 1, wobei der zu rekonstruierende Genotyp folgendermaßen ist:

a. das *FMR1*-Gen oder ein Fragment davon, wobei die Probe eine *FMR1*-Nukleinsäure oder ein Fragment davon umfasst, und wobei die Parameterinformation das Bestimmen der Gesamtlänge von zumindest einem CGG-Repeat-Bereich in der *FMR1*-Nukleinsäure oder Fragment, die Entfernung vom Beginn des zumindest einen CGG-Repeat-Bereichs zu einer oder mehreren AGG-Unterbrechungen in dem Bereich und die Entfernung vom Ende des zumindest einen CGG-Repeat-Bereichs zu einer oder mehrere AGG-Unterbrechungen in dem Bereich umfasst, oder
b. das *FMR2*-Gen oder ein Fragment davon, wobei die Probe eine *FMR2*-Nukleinsäure oder ein Fragment davon umfasst, und wobei die Parameterinformation das Bestimmen der Gesamtlänge von zumindest einem CCG-Repeat-Bereich in der *FMR2*-Nukleinsäure oder Fragment, die Entfernung vom Beginn des zumindest einen CCG-Repeat-Bereichs zu einer oder mehreren AGG-Unterbrechungen in dem Bereich und die Entfernung vom Ende des zumindest einen CCG-Repeat-Bereichs zu einer oder mehreren AGG-Unterbrechungen in dem Bereich umfasst.

**6.** Verfahren nach Anspruch 5, ferner umfassend das Verwenden des rekonstruierten Genotyps, um einen Genotyp nachzuweisen, der mit einer *FMR1*- oder *FMR2*-Störung oder einem Phänotyp bei einem Patienten assoziiert ist, oder um ein Risiko von einer *FMR1*- oder *FMR2*-Störung oder Phänotyp bei den Nachkommen des Patienten nachzuweisen.

**7.** Verfahren nach Anspruch 6, wobei die *FMR1*- oder *FMR2*-Störung das Fragiles-X-Syndrom (FXS), das Fragiles-X-Syndrom E (FRAXE), das Fragiles-X-assoziierte Tremor / Ataxie-Syndrom (FXTAS), die Fragiles-X-assoziierte primäre Ovarialinsuffizienz (FXPOI) ist oder Dopamin-responsiver Parkinsonismus ist.

**8.** Apparat zur automatisierten Rekonstruktion eines Genotyps, umfassend

a. einen Prozessor; und
b. eine Speichervorrichtung, die kommunikativ an den Prozessor gekoppelt ist, wobei die Speichervorrichtung darin maschinenausführbare Anweisungen gespeichert hat, die, wenn sie von dem Prozessor ausgeführt werden, den Prozessor veranlassen,

i. Parameterinformationen für eine Nukleinsäure zu empfangen, wobei die Nukleinsäure zumindest einen GC-reichen Nukleotid-Repeat-Bereich aufweist, der eine oder mehrere Unterbrechungen umfasst; und wobei die Parameterinformation die Gesamtlänge des zumindest einen GC-reichen Nukleotid-Repeat-Bereichs, die Entfernung vom Beginn des zumindest einen GC-reichen Nukleotid-Repeat-Bereichs zu der einen oder mehreren Unterbrechungen in dem Bereich und die Entfernung vom Ende des zumindest einen GC-reichen Nukleotid-Repeat-Bereichs zu der einen oder mehreren Unterbrechungen in dem Bereich umfasst;
ii. einen Set von potenziellen Genotypen zu erzeugen, die alle möglichen Anordnungen von der einen oder mehreren Unterbrechungen in dem Bereich umfassen, durch Durchführen einer automatisierten umfassenden Aufzählung der Parameterinformation;
iii. den Satz von potenziellen Genotypen auszuwerten, um einen Lösungsgenotypen herzustellen, der alle Parameterinformationen erfüllt; und

iv. den Lösungsgenotyp auf der Speichervorrichtung zu speichern.

**9.** Apparat nach Anspruch 8, ferner umfassend einen Monitor, der kommunikativ an den Prozessor und Speichervorrichtung gekoppelt ist, wobei die maschinenausführbaren Anweisungen, die in der Speichervorrichtung gespeichert sind, den Prozessor anweisen, den Lösungsgenotypen auf dem Monitor anzuzeigen.

**10.** Apparat nach Anspruch 8, ferner umfassend einen Drucker, der kommunikativ an den Prozessor und Speichervorrichtung gekoppelt ist, wobei die maschinenausführbaren Anweisungen, die in der Speichervorrichtung gespeichert sind, den Prozessor anweisen, den Lösungsgenotypen mit dem Drucker zu drucken.

**11.** Maschinenlesbares Medium, das maschinenausführbare Anweisungen umfasst, die, wenn sie von einem Prozessor ausgeführt werden, den Prozessor dazu veranlassen,

a. Parameterinformation für eine Nukleinsäure zu empfangen, wobei die Nukleinsäure zumindest einen GC-reichen Nukleotid-Repeat-Bereich aufweist, der eine oder mehrere Unterbrechungen umfasst; und wobei die Parameterinformation die Gesamtlänge des zumindest einen GC-reichen Nukleotid-Repeat-Bereichs, die Entfernung vom Beginn des zumindest einen GC-reichen Nukleotid-Repeat-Bereichs zu der einen oder mehreren Unterbrechungen in dem Bereich und die Entfernung vom Ende des zumindest einen GC-reichen Nukleotid-Repeat-Bereichs zu der einen oder mehreren Unterbrechungen in dem Bereich umfasst;
b. einen Satz potenzieller Genotypen zu erzeugen, die alle möglichen Anordnungen der einen oder mehreren Unterbrechungen in dem Bereich umfassen, durch Durchführen einer automatisierten umfassenden Aufzählung der Parameterinformation
c. den Satz potenzieller Genotypen auszuwerten, um einen Lösungsgenotypen herzustellen, der alle Parameterinformationen erfüllt; und
d. den Lösungsgenotypen auf einer Speichervorrichtung zu speichern.

**12.** Maschinenlesbares Medium nach Anspruch 11, wobei die maschinenausführbaren Anweisungen den Prozessor anweisen, den Lösungsgenotypen auf einem Monitor anzuzeigen.

**13.** Maschinenlesbares Medium nach Anspruch 11, wobei die maschinenausführbaren Anweisungen den Prozessor anweisen, den Lösungsgenotypen mit einem Drucker zu drucken.

**14.** Kit, umfassend den Apparat nach einem der Ansprüche 8-10 oder maschinenlesbares Medium nach einem der Ansprüche 11-13 und Anweisungen zur Verwendung des Apparates oder des maschinenlesbaren Mediums zur Rekonstruktion eines Genotyps.

**15.** Kit nach Anspruch 14, wobei das Kit verwendet wird, um einen Genotyp nachzuweisen, der mit einer *FMR1*- oder FMR2-Störung oder Phänotyp eines Patienten assoziiert ist, oder um ein Risiko von einer *FMR1*- oder *FMR2*-Störung oder Phänotyp bei den Nachkommen des Patienten nachzuweisen.

## Revendications

**1.** Procédé de reconstruction automatisée d'un génotype, comprenant

a. la fourniture d'un acide nucléique isolé à partir d'un échantillon d'un patient, l'acide nucléique ayant au moins une région répétée de nucléotides riche en GC comprenant une ou plusieurs interruptions ;
b. la détermination d'informations de paramètres pour l'acide nucléique, la détermination de l'information de paramètres comprenant la mesure de la longueur totale de la ou des régions répétées de nucléotides riches en GC, la distance entre le début de la ou des régions répétées de nucléotides riches en GC et la ou les interruptions dans la région, et la distance entre la fin de la ou des régions répétées de nucléotides riches en GC et la ou les interruptions dans la région ; et
c. l'utilisation d'un appareil pour réaliser l'énumération exhaustive automatisée sur les informations de paramètres pour produire un génotype reconstruit, l'énumération exhaustive comprenant

i) l'utilisation de la longueur totale de la ou des régions répétées riches en nucléotides GC et soit la distance entre le début de la ou des régions répétées de nucléotides riches en GC et la ou les interruptions dans la région soit la distance entre la fin de la ou des régions répétées de nucléotides riches en GC et la ou les interruptions dans la région pour produire une série de génotypes potentiels comprenant tous les arrangements possibles de la ou des interruptions dans la région ; et
ii) l'évaluation de la série de génotypes potentiels pour déterminer un génotype solution qui satisfait à toutes les informations de paramètres.

**2.** Procédé de la revendication 1, dans lequel les informations de paramètres sont déterminées par réaction en chaîne à la polymérase.

**3.** Procédé de la revendication 1, dans lequel l'appareil comprend un processeur et un dispositif de mémoire couplé en communication au processeur, le dispositif de mémoire ayant en stockage des instructions exécutables par machine qui, lorsqu'elles sont exécutées par le processeur, font recevoir au processeur des informations de paramètres et exécuter une analyse d'énumération exhaustive.

**4.** Procédé de la revendication 1, comprenant en outre la confirmation du génotype reconstruit par comparaison manuelle du génotype aux informations de paramètres en effectuant une digestion de restriction, ou par séquençage de l'acide nucléique ayant au moins une région répétée de nucléotides riche en GC.

**5.** Procédé de la revendication 1, dans lequel le génotype étant reconstruit est :

a. le gène *FMR1* ou un fragment de celui-ci, l'échantillon comprenant un acide nucléique *FMR1* ou un fragment de celui-ci, et les informations de paramètres comprenant la détermination de la longueur totale d'au moins une région répétée CGG dans l'acide nucléique ou fragment *FMR1,* la distance entre le début de la ou des régions répétées CGG et la ou les interruptions AGG dans la région, et la distance entre la fin de la ou des régions répétées CGG à la ou les interruptions AGG dans la région, ou
b. le gène *FMR2* ou un fragment de celui-ci, l'échantillon comprenant un acide nucléique *FMR2* ou un fragment de celui-ci, et les informations de paramètres comprenant la détermination de la longueur totale d'au moins une région répétée CCG dans l'acide nucléique ou le fragment *FMR2,* la distance entre le début de la ou des régions répétées CCG et la ou les interruptions AGG dans la région, et la distance entre la fin de la ou des régions répétées CCG à la ou les interruptions AGG interruptions dans la région.

**6.** Procédé de la revendication 5, comprenant en outre l'utilisation du génotype reconstruit pour détecter un génotype associé à un trouble ou phénotype *FMR1* ou *FMR2* chez un patient, ou pour détecter un risque de trouble ou de phénotype *FMR1* ou *FMR2* dans la descendance du patient.

**7.** Procédé de la revendication 6, dans lequel le trouble *FMR1* ou *FMR2* est un syndrome du X fragile (FXS), un syndrome E du X fragile (FRAXE), un syndrome de tremblements/ataxie associé au X fragile (FXTAS), une insuffisance ovarienne primaire liée au X fragile (FXPOI), ou un parkinsonisme répondant à la dopamine.

**8.** Appareil pour la reconstruction automatisée d'un génotype, comprenant

a. un processeur ; et
b. un dispositif de mémoire couplé en communication au processeur, le dispositif de mémoire ayant en stockage des instructions exécutables par machine qui, lorsqu'elles sont exécutées par le processeur, font que le processeur

i. reçoit des informations de paramètres pour un acide nucléique, l'acide nucléique ayant au moins une région répétée de nucléotides riche en GC comprenant une ou plusieurs interruptions ; et les informations de paramètres comprenant la longueur totale de la ou des régions répétées de nucléotides riches en GC, la distance entre le début de la ou des régions répétées de nucléotides riches en GC et la ou les interruptions dans la région, et la distance entre la fin de la ou des régions répétées de nucléotides riches en GC et la ou les interruptions dans la région ;
ii. produit une série de génotypes potentiels comprenant tous les arrangements possibles de la ou des interruptions dans la région en effectuant une énumération exhaustive automatisée sur les informations de paramètres ;
iii. évalue la série de génotypes potentiels pour produire un génotype solution qui satisfait à toutes les informations de paramètres ; et
iv. stocke le génotype solution sur le dispositif de mémoire.

**9.** Appareil de la revendication 8, comprenant en outre un moniteur relié en communication au processeur et au dispositif de mémoire, dans lequel les instructions exécutables par machine stockées sur le dispositif de mémoire commandent au processeur d'afficher le génotype solution sur le moniteur.

**10.** Appareil de la revendication 8, comprenant en outre une imprimante couplée en communication au processeur et au dispositif de mémoire, dans lequel les instructions exécutables par machine stockées sur le dispositif de mémoire commandent au processeur d'afficher le génotype solution sur l'imprimante.

**11.** Support lisible par machine comprenant des instructions utilisables par une machine qui, lorsqu'elles sont exécutées par un processeur, font que le processeur

a. reçoit des informations de paramètres pour un acide nucléique, l'acide nucléique ayant au moins une région répétée de nucléotides riche en GC comprenant une ou plusieurs interruptions ; et les informations de paramètres comprenant la longueur totale de la ou des régions répétées de nucléotides riches en GC, la distance entre le début de la ou des régions répétées de nucléotides riches en GC et la ou les interruptions dans la région, et la distance entre la fin de la ou des régions répétées de nucléotides riches en GC et la ou les interruptions dans la région ;
b. produit une série de génotypes potentiels comprenant tous les arrangements possibles de la ou des interruptions dans la région en effectuant une énumération exhaustive automatisée sur les informations de paramètres
c. évalue la série de génotypes potentiels pour produire un génotype solution qui satisfait à toutes les informations de paramètres ; et
d. stocke le génotype solution sur un dispositif de mémoire.

**12.** Support lisible par machine de la revendication 11, dans lequel les instructions exécutables par la machine commandent au processeur d'afficher le génotype solution sur un moniteur.

**13.** Support lisible par machine de la revendication 11, dans lequel les instructions exécutables par la machine commandent au processeur d'imprimer le génotype solution sur une imprimante.

**14.** Kit comprenant l'appareil d'une quelconque des revendications 8 à 10, ou support lisible par machine d'une quelconque des revendications 11 à 13, et instructions d'utilisation de l'appareil ou du support lisible par machine pour reconstruire un génotype.

**15.** Kit de la revendication 14, le kit étant utilisé pour détecter un génotype associé à un trouble ou phénotype *FMR1* ou *FMR2* chez un patient, ou pour détecter un risque de trouble ou de phénotype *FMR1* ou *FMR2* dans la descendance du patient.

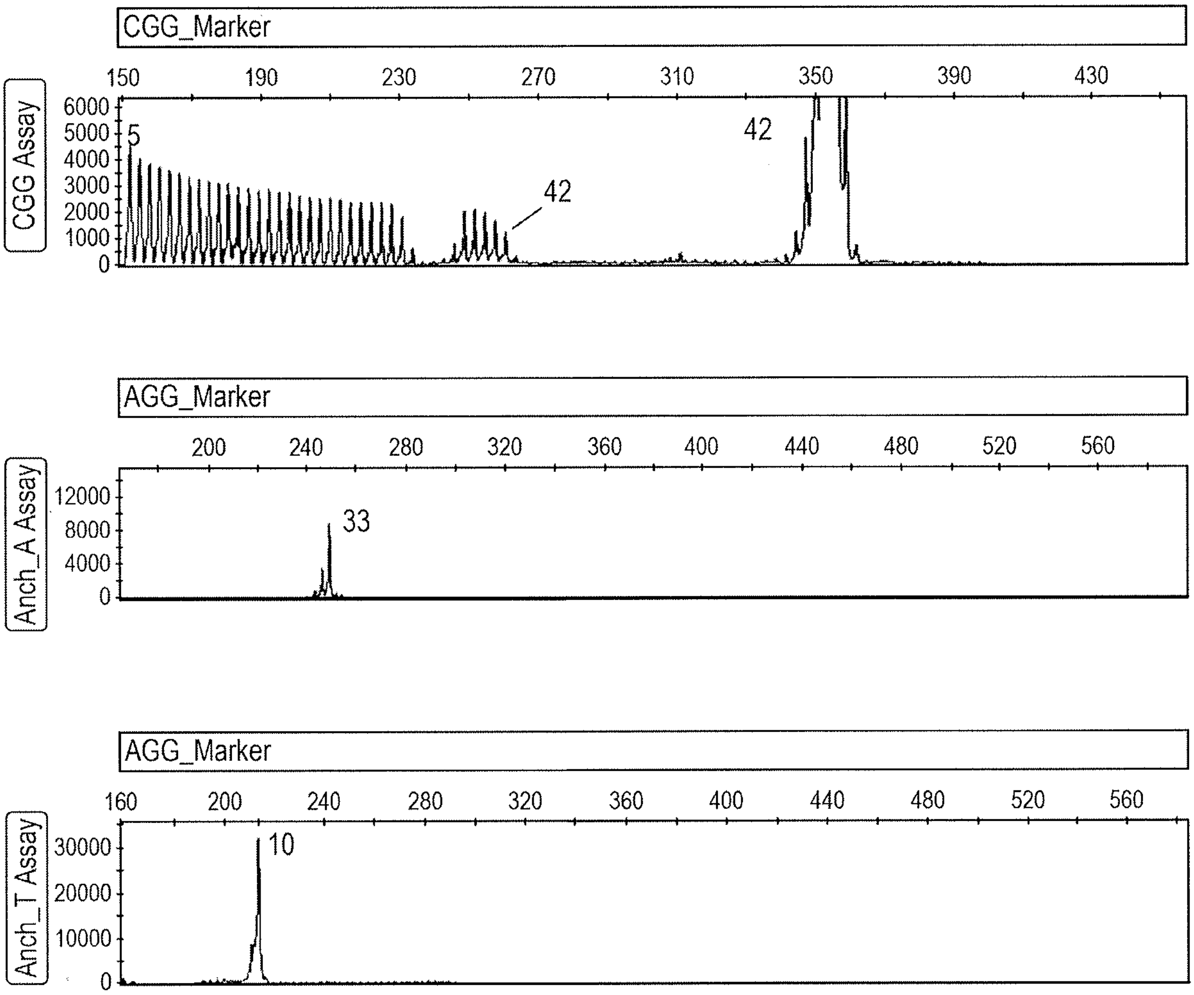
CGG_Marker
150
190
230
270
310
350
390
430
CGG Assay
6000
5000
4000
3000
2000
1000
0
5
42
42
AGG_Marker
200
240
280
320
360
400
440
480
520
560
Anch_A Assay
12000
8000
4000
0
33
AGG_Marker
160
200
240
280
320
360
400
440
480
520
560
Anch_T Assay
30000
20000
10000
0
10

FIG. 1

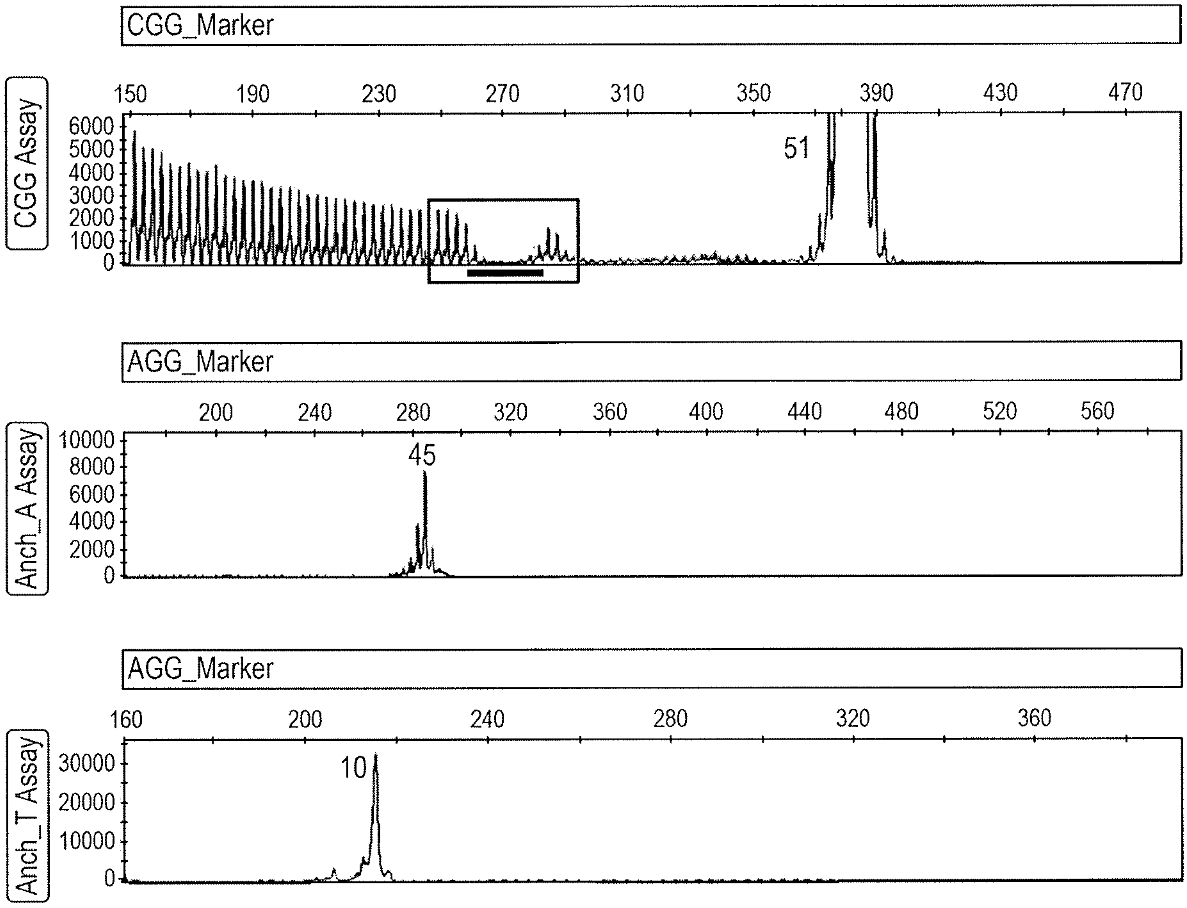
CGG_Marker
CGG Assay
150
190
230
270
310
350
390
430
470
6000
5000
4000
3000
2000
1000
0
51
AGG_Marker
Anch_A Assay
200
240
280
320
360
400
440
480
520
560
10000
8000
6000
4000
2000
0
45
AGG_Marker
Anch_T Assay
160
200
240
280
320
360
30000
20000
10000
0
10

FIG. 2

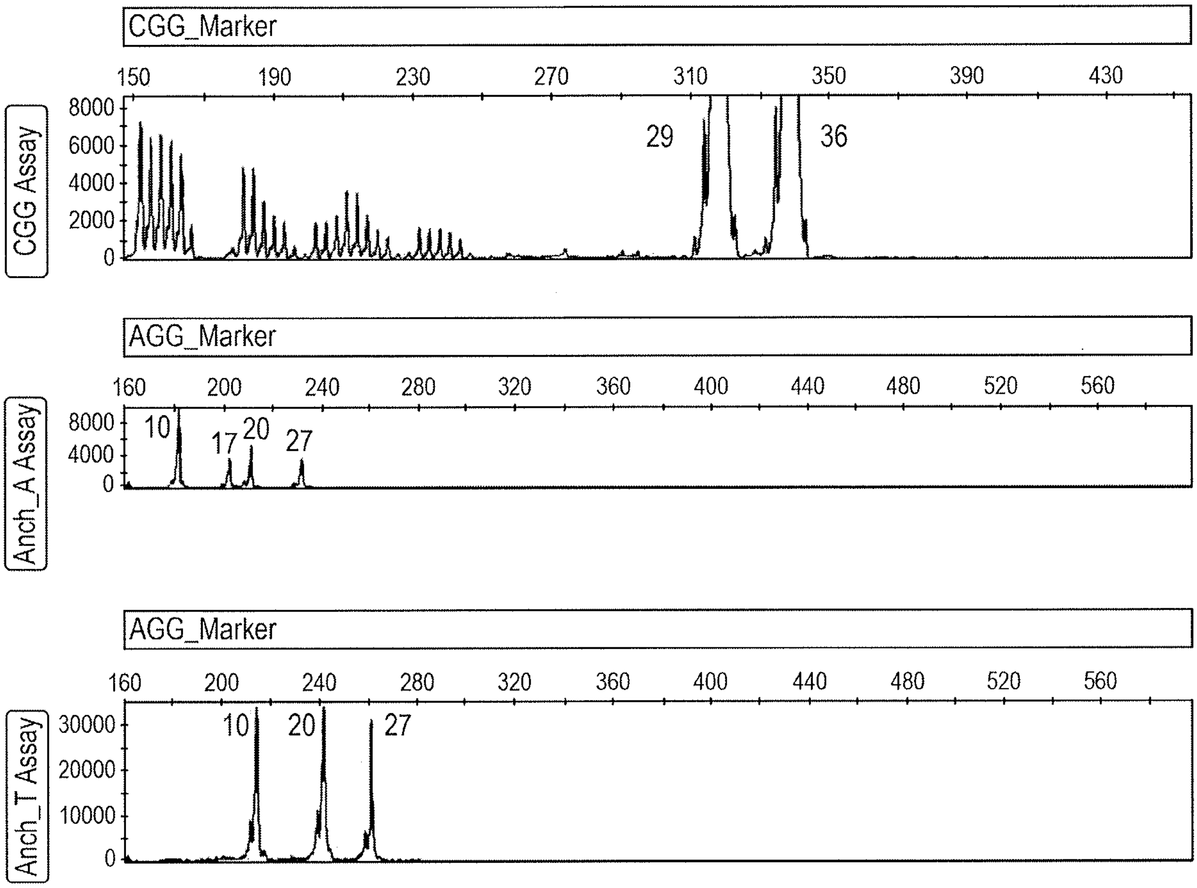

CGG_Marker
150
190
230
270
310
350
390
430
CGG Assay
8000
6000
4000
2000
0
29
36
AGG_Marker
160
200
240
280
320
360
400
440
480
520
560
Anch_A Assay
8000
4000
0
10
17
20
27
AGG_Marker
160
200
240
280
320
360
400
440
480
520
560
Anch_T Assay
30000
20000
10000
0
10
20
27


FIG. 3

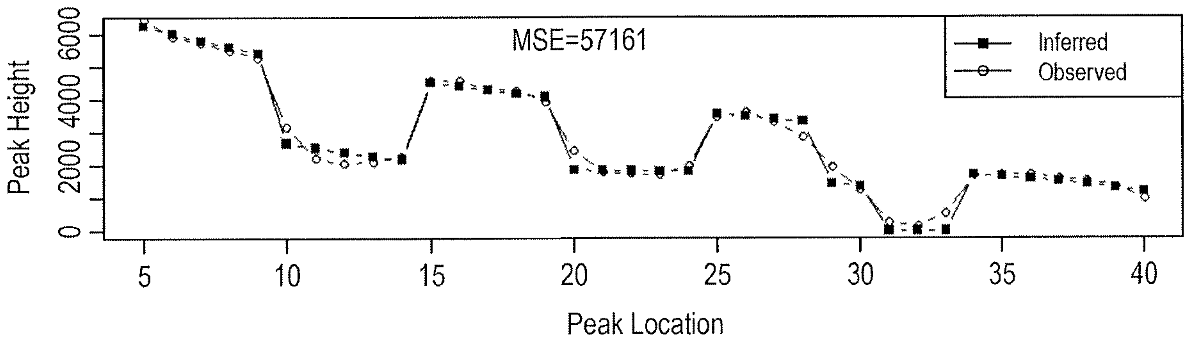
MSE=57161
Inferred
Observed
Peak Height
0 2000 4000 6000
5 10 15 20 25 30 35 40
Peak Location

FIG. 4A

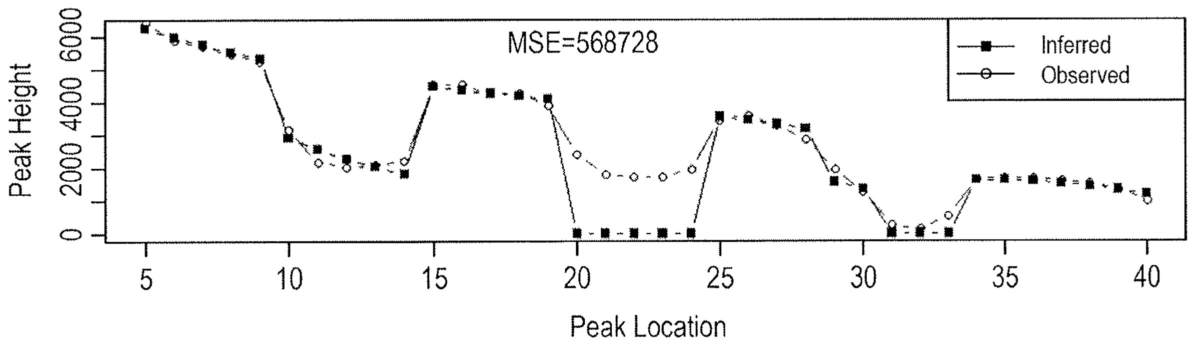
MSE=568728
Inferred
Observed
Peak Height
0 2000 4000 6000
5 10 15 20 25 30 35 40
Peak Location

FIG. 4B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61674167 A
- US 20100209970 A
- US 20100243451 A
- US 20120107824 A
- US 8409805 B
- US 371306 A
- WO 61674167 A


### Non-patent literature cited in the description


- *J. Mol. Diag.,* 2008, vol. 10 (6), 496-501
- *Genome Res.,* 1996, vol. 6 (7), 633-8
- *J. Mol. Diag.,* 2006, vol. 8, 544-550
- *Am. J. Med. Genet.,* 1994, vol. 51 (4), 527-34
- **EICHLER et al.** *Nat. Genet.,* 1994, vol. 8, 88-94
- **NOLIN et al.** *Am. J. Hum. Genet.,* 2003, vol. 72, 454-64
- **YRIGOLLEN et al.** *Genet. Med.*
- **LIANGJING CHEN et al.** An Information-Rich CGG Repeat Primed PCR that detects the full range of fragile X expanded alleles and minimizes the need for southern blot analysis. *Journal of Molecular Diagnosis,* 01 September 2010, vol. 12 (5
- **TASSONE et al.** *J Mol Diagn.,* 2008, vol. 10 (1), 43-49
- **CHEN et al.** *J Mol Diagn.,* 2010, vol. 12 (5), 589-600
- **YRIGOLLEN et al.** *PLoS One,* 2011, vol. 6 (7), e21728
- *Nat Genet,* May 1996, vol. 13 (1), 105-8
- *Nat Genet,* May 1996, vol. 13 (1), 109-13
- **NOLIN et al.** *Am. J. Med. Genet. Part A,* 2013, vol. 9999, 1-8
- **TASSONE et al.** *J. Mol. Diagn.,* 2008, vol. 10, 43-49
- **CHEN et al.** *J. Mol. Diagn.,* 2010, vol. 12, 589-600
- **FILIPOVIC-SADIC et al.** *Clin. Chem.,* 2010, vol. 56, 399-408

SEQUENCE LISTING

<110> ASURAGEN, INC.

<120> COMPREHENSIVE FMR1 GENOTYPING

<130> 10256.0039-00304

<140>
<141>

<150> 61/674,167
<151> 2012-07-20

<160> 66

<170> PatentIn version 3.5

<210> 1
<211> 22
<212> DNA
<213> Artificial Sequence

<220>
<221> source
<223> /note="Description of Artificial Sequence: Synthetic primer"

<400> 1
cggtggaggg ccgcctctga gc 22

<210> 2
<211> 25
<212> DNA
<213> Artificial Sequence

<220>
<221> source
<223> /note="Description of Artificial Sequence: Synthetic primer"

<400> 2
caggcgctca gctccgtttc ggttt 25

<210> 3
<211> 25
<212> DNA
<213> Artificial Sequence

<220>
<221> source
<223> /note="Description of Artificial Sequence: Synthetic primer"

<400> 3
cagtcaggcg ctcagctccg tttcg 25

<210> 4
<211> 24
<212> DNA
<213> Artificial Sequence

<220>
<221> source
<223> /note="Description of Artificial Sequence: Synthetic primer"

<400> 4
tccggtggag ggccgcctct gagc 24

<210> 5
<211> 35
<212> DNA
<213> Artificial Sequence

<220>
<221> source
<223> /note="Description of Artificial Sequence: Synthetic primer"

<400> 5

ggttcggcct cagtcaggcg ctcagctccg tttcg 35

<210> 6
<211> 36
<212> DNA
<213> Artificial Sequence

<220>
<221> source
<223> /note="Description of Artificial Sequence: Synthetic primer"

<400> 6
gggttcggcc tcagtcaggc gctcagctcc gtttcg 36

<210> 7
<211> 26
<212> DNA
<213> Artificial Sequence

<220>
<221> source
<223> /note="Description of Artificial Sequence: Synthetic primer"

<400> 7
gcgggccggg ggttcggcct cagtca 26

<210> 8
<211> 25
<212> DNA
<213> Artificial Sequence

<220>
<221> source
<223> /note="Description of Artificial Sequence: Synthetic primer"

<400> 8
cagcgggccg ggggttcggc ctcag 25

<210> 9
<211> 25
<212> DNA
<213> Artificial Sequence

<220>
<221> source
<223> /note="Description of Artificial Sequence: Synthetic primer"

<400> 9
gcagcgggcc gggggttcgg cctca 25

<210> 10
<211> 25
<212> DNA
<213> Artificial Sequence

<220>
<221> source
<223> /note="Description of Artificial Sequence: Synthetic primer"

<400> 10
gggccggggg ttcggcctca gtcag 25

<210> 11
<211> 26
<212> DNA
<213> Artificial Sequence

<220>
<221> source
<223> /note="Description of Artificial Sequence: Synthetic primer"

<400> 11
ggggttcggc ctcagtcagg cgctca 26

<210> 12
<211> 27
<212> DNA
<213> Artificial Sequence

<220>
<221> source
<223> /note="Description of Artificial Sequence: Synthetic primer"

<400> 12
ggggttcggc ctcagtcagg cgctcag 27

<210> 13
<211> 30
<212> DNA
<213> Artificial Sequence

<220>
<221> source
<223> /note="Description of Artificial Sequence: Synthetic primer"

<400> 13
ggcgctcagc tccgtttcgg tttcacttcc 30

<210> 14
<211> 28
<212> DNA
<213> Artificial Sequence

<220>
<221> source
<223> /note="Description of Artificial Sequence: Synthetic primer"

<400> 14
tcaggcgctc agctccgttt cggtttca 28

<210> 15
<211> 26
<212> DNA
<213> Artificial Sequence

<220>
<221> source

<223> /note="Description of Artificial Sequence: Synthetic primer"

<400> 15
cacttccggt ggagggccgc ctctga 26

<210> 16
<211> 25
<212> DNA
<213> Artificial Sequence

<220>
<221> source
<223> /note="Description of Artificial Sequence: Synthetic primer"

<400> 16
ttccggtgga gggccgcctc tgagc 25

<210> 17
<211> 25
<212> DNA
<213> Artificial Sequence

<220>
<221> source
<223> /note="Description of Artificial Sequence: Synthetic primer"

<400> 17
cgcacttcca ccaccagctc ctcca 25

<210> 18
<211> 21
<212> DNA
<213> Artificial Sequence

<220>
<221> source
<223> /note="Description of Artificial Sequence: Synthetic primer"

<400> 18
ggagcccgcc cccgagaggt g 21

<210> 19
<211> 21
<212> DNA
<213> Artificial Sequence

<220>
<221> source
<223> /note="Description of Artificial Sequence: Synthetic primer"

<400> 19
gggagcccgc ccccgagagg t 21

<210> 20
<211> 26
<212> DNA
<213> Artificial Sequence

<220>
<221> source
<223> /note="Description of Artificial Sequence: Synthetic primer"

<400> 20
cgcacttcca ccaccagctc ctccat 26

<210> 21
<211> 23
<212> DNA
<213> Artificial Sequence

<220>
<221> source
<223> /note="Description of Artificial Sequence: Synthetic primer"

<400> 21
cgggagcccg cccccgagag gtg 23

<210> 22
<211> 23
<212> DNA
<213> Artificial Sequence

<220>
<221> source
<223> /note="Description of Artificial Sequence: Synthetic primer"

<400> 22
ccgggagccc gcccccgaga ggt 23

<210> 23
<211> 24
<212> DNA
<213> Artificial Sequence

<220>
<221> source
<223> /note="Description of Artificial Sequence: Synthetic primer"

<400> 23
ccgggagccc gcccccgaga ggtg 24

<210> 24
<211> 26
<212> DNA
<213> Artificial Sequence

<220>
<221> source
<223> /note="Description of Artificial Sequence: Synthetic primer"

<400> 24
cgccgggagc ccgcccccga gaggtg 26

<210> 25
<211> 26
<212> DNA

<213> Artificial Sequence

<220>
<221> source
<223> /note="Description of Artificial Sequence: Synthetic primer"

<400> 25
gcgccgggag cccgcccccg agaggt 26

<210> 26
<211> 25
<212> DNA
<213> Artificial Sequence

<220>
<221> source
<223> /note="Description of Artificial Sequence: Synthetic primer"

<400> 26
cgccgggagc ccgcccccga gaggt 25

<210> 27
<211> 28
<212> DNA
<213> Artificial Sequence

<220>
<221> source
<223> /note="Description of Artificial Sequence: Synthetic primer"

<400> 27
gcgccattgg agccccgcac ttccacca 28

<210> 28
<211> 25
<212> DNA
<213> Artificial Sequence

<220>
<221> source
<223> /note="Description of Artificial Sequence: Synthetic primer"

<400> 28
gcgccattgg agccccgcac ttcca 25

<210> 29
<211> 25
<212> DNA
<213> Artificial Sequence

<220>
<221> source
<223> /note="Description of Artificial Sequence: Synthetic primer"

<400> 29
agcgccattg gagccccgca cttcc 25

<210> 30

<211> 25
<212> DNA
<213> Artificial Sequence

<220>
<221> source
<223> /note="Description of Artificial Sequence: Synthetic primer"

<400> 30
cgccattgga gccccgcact tccac 25

<210> 31
<211> 25
<212> DNA
<213> Artificial Sequence

<220>
<221> source
<223> /note="Description of Artificial Sequence: Synthetic primer"

<400> 31
ttggagcccc gcacttccac cacca 25

<210> 32
<211> 28
<212> DNA
<213> Artificial Sequence

<220>
<221> source
<223> /note="Description of Artificial Sequence: Synthetic primer"

<400> 32
agccccgcac ttccaccacc agctcctc 28

<210> 33
<211> 28
<212> DNA
<213> Artificial Sequence

<220>
<221> source
<223> /note="Description of Artificial Sequence: Synthetic primer"

<400> 33
gagccccgca cttccaccac cagctcct 28

<210> 34
<211> 28
<212> DNA
<213> Artificial Sequence

<220>
<221> source
<223> /note="Description of Artificial Sequence: Synthetic primer"

<400> 34
cattggagcc ccgcacttcc accaccag 28

<210> 35
<211> 30
<212> DNA
<213> Artificial Sequence

<220>
<221> source
<223> /note="Description of Artificial Sequence: Synthetic primer"

<400> 35
cccgcacttc caccaccagc tcctccatct 30

<210> 36
<211> 30
<212> DNA
<213> Artificial Sequence

<220>
<221> source
<223> /note="Description of Artificial Sequence: Synthetic primer"

<400> 36
tagaaagcgc cattggagcc ccgcacttcc 30

<210> 37
<211> 26
<212> DNA
<213> Artificial Sequence

<220>
<221> source
<223> /note="Description of Artificial Sequence: Synthetic primer"

<400> 37
aagcgccatt ggagccccgc acttcc 26

<210> 38
<211> 36
<212> DNA
<213> Artificial Sequence

<220>
<221> source
<223> /note="Description of Artificial Sequence: Synthetic primer"

<400> 38
tcaggcgctc agctccgttt cggtttcact tccggt 36

<210> 39
<211> 36
<212> DNA
<213> Artificial Sequence

<220>
<221> source
<223> /note="Description of Artificial Sequence: Synthetic primer"

<400> 39

agcgtctact gtctcggcac ttgcccgccg ccgccg 36

<210> 40
<211> 28
<212> DNA
<213> Artificial Sequence

<220>
<221> source
<223> /note="Description of Artificial Sequence: Synthetic primer"

<400> 40
tcaggcgctc agctccgttt cggtttca 28

<210> 41
<211> 43
<212> DNA
<213> Artificial Sequence

<220>
<221> source
<223> /note="Description of Artificial Sequence: Synthetic primer"

<400> 41
tcaggcgctc agctccgttt cggtttcacg gcggcggcgg cgg 43

<210> 42
<211> 16
<212> DNA
<213> Artificial Sequence

<220>
<221> source
<223> /note="Description of Artificial Sequence: Synthetic oligonucleotide"

<400> 42
cggcggcggc ggcgga 16

<210> 43
<211> 41
<212> DNA
<213> Artificial Sequence

<220>
<221> source
<223> /note="Description of Artificial Sequence: Synthetic primer"

<400> 43
aagcgccatt ggagccccgc acttccccgc cgccgccgcc g 41

<210> 44
<211> 44
<212> DNA
<213> Artificial Sequence

<220>
<221> source
<223> /note="Description of Artificial Sequence: Synthetic primer"

<400> 44
tcaggcgctc agctccgttt cggtttcacg gcggcggcgg cgga 44

<210> 45
<211> 41
<212> DNA
<213> Artificial Sequence

<220>
<221> source
<223> /note="Description of Artificial Sequence: Synthetic primer"

<400> 45
aagcgccatt ggagccccgc acttccccgc cgccgccgcc t 41

<210> 46
<211> 15
<212> DNA
<213> Artificial Sequence

<220>
<221> source
<223> /note="Description of Artificial Sequence: Synthetic oligonucleotide"

<400> 46
tccgccgccg ccgcc 15

<210> 47
<211> 69
<212> DNA
<213> Artificial Sequence

<220>
<221> source
<223> /note="Description of Artificial Sequence: Synthetic oligonucleotide"

<400> 47

cggcggcggc ggaggcggcg gcggcggcgg cggcggcggc ggcggcggcg gaggcggcgg 60

cggcggcgg 69

<210> 48
<211> 87
<212> DNA
<213> Artificial Sequence

<220>
<221> source
<223> /note="Description of Artificial Sequence: Synthetic oligonucleotide"

<400> 48

cggcggcggc ggcggcggcg gcggcggcgg cggcggcggc ggcggcggcg gcggcggagg 60

cggcggcggc ggcggcggcg gcggcgg 87

<210> 49
<211> 156
<212> DNA
<213> Artificial Sequence

<220>
<221> source
<223> /note="Description of Artificial Sequence: Synthetic polynucleotide"

<400> 49

cggcggcggc ggcggcggcg gcggcggcgg aggcggcggc ggcggcggcg gcggcggcgg 60

cggcggcggc ggcggcggcg gcggcggcgg cggcggcggc ggcggcggcg gcggcggcgg 120

cggcggcggc ggcggcggcg gcggcggcgg cggcgg 156

<210> 50
<211> 87
<212> DNA
<213> Artificial Sequence

<220>
<221> source
<223> /note="Description of Artificial Sequence: Synthetic oligonucleotide"

<400> 50

cggcggcggc ggcggcggcg gcggcggcgg cggcggcggc ggcggcggcg gcggcggcgg 60

cggcggcggc ggcggcggcg gcggcgg 87

<210> 51
<211> 87
<212> DNA
<213> Artificial Sequence

<220>
<221> source
<223> /note="Description of Artificial Sequence: Synthetic oligonucleotide"

<400> 51

cggcggcggc ggcggcggcg gcggcggcgg aggcggcggc ggcggcggcg gcggcggcgg 60

cggcggcggc ggcggcggcg gcggcgg 87

<210> 52
<211> 87
<212> DNA
<213> Artificial Sequence

<220>
<221> source
<223> /note="Description of Artificial Sequence: Synthetic oligonucleotide"

<400> 52

cggcggcggc ggcggcggcg gcggcggcgg aggcggcggc ggcggcggcg gcggcggagg 60

cggcggcggc ggcggcggcg gcggcgg 87

<210> 53
<211> 156
<212> DNA
<213> Artificial Sequence

<220>
<221> source
<223> /note="Description of Artificial Sequence: Synthetic polynucleotide"

<400> 53

cggcggcggc ggcggcggcg gcggcggcgg cggcggcggc ggcggcggcg gcggcggcgg 60

cggcggcggc ggcggcggcg gcggcggcgg cggcggcggc ggcggcggcg gcggcggcgg 120

cggcggcggc ggcggcggcg gcggcggcgg cggcgg 156

<210> 54
<211> 156
<212> DNA
<213> Artificial Sequence

<220>
<221> source
<223> /note="Description of Artificial Sequence: Synthetic polynucleotide"

<400> 54

cggcggcggc ggcggcggcg gcggcggcgg cggcggcggc ggcggcggcg gcggcggagg 60

cggcggcggc ggcggcggcg gcggcggcgg cggcggcggc ggcggcggcg gcggcggcgg 120

cggcggcggc ggcggcggcg gcggcggcgg cggcgg 156

<210> 55
<211> 156
<212> DNA
<213> Artificial Sequence

<220>
<221> source
<223> /note="Description of Artificial Sequence: Synthetic polynucleotide"

<400> 55

cggcggcggc ggcggcggcg gcggcggcgg aggcggcggc ggcggcggcg gcggcggagg 60

cggcggcggc ggcggcggcg gcggcggcgg cggcggcggc ggcggcggcg gcggcggcgg 120

cggcggcggc ggcggcggcg gcggcggcgg cggcgg 156

<210> 56

<211> 126
<212> DNA
<213> Artificial Sequence

<220>
<221> source
<223> /note="Description of Artificial Sequence: Synthetic polynucleotide"

<400> 56

cggcggcggc ggcggcggcg gcggcggagg cggcggcggc ggcggcggcg gcggcggcgg 60

cggcggcggc ggcggcggcg gcggcggcgg cggcggcggc ggcggcggcg gcggcggcgg 120

cggcgg 126

<210> 57
<211> 15
<212> DNA
<213> Artificial Sequence

<220>
<221> source
<223> /note="Description of Artificial Sequence: Synthetic oligonucleotide"

<400> 57
ccgccgccgc cgcct 15

<210> 58
<211> 153
<212> DNA
<213> Artificial Sequence

<220>
<221> source
<223> /note="Description of Artificial Sequence: Synthetic polynucleotide"

<400> 58

cggcggcggc ggcggcggag gcggcggagg cggcggcggc ggcggcggcg gcggcggcgg 60

cggcggcggc ggcggcggcg gcggcggcgg cggcggcggc ggcggcggcg gcggcggcgg 120

cggcggcggc ggcggcggcg gcggcggcgg cgg 153

<210> 59
<211> 15
<212> DNA
<213> Artificial Sequence

<220>
<221> source
<223> /note="Description of Artificial Sequence: Synthetic oligonucleotide"

<400> 59
cggcggcggc ggcgg 15

<210> 60

<211> 87
<212> DNA
<213> Artificial Sequence

<220>
<221> source
<223> /note="Description of Artificial Sequence: Synthetic oligonucleotide"

<400> 60

cggcggcggc ggcggcggcg gcggcggagg cggcggcggc ggcggcggcg gcggcggagg 60

cggcggcggc ggcggcggcg gcggcgg 87

<210> 61
<211> 108
<212> DNA
<213> Artificial Sequence

<220>
<221> source
<223> /note="Description of Artificial Sequence: Synthetic polynucleotide"

<400> 61

cggcggcggc ggcggcggcg gcggcggagg cggcggcggc ggcggcggcg gcggcggagg 60

cggcggcggc ggcggcggag gcggcggcgg cggcggcggc ggcggcgg 108

<210> 62
<211> 87
<212> DNA
<213> Artificial Sequence

<220>
<221> source
<223> /note="Description of Artificial Sequence: Synthetic oligonucleotide"

<400> 62

cggcggcggc ggcggcggcg gcggcggagg cggcggcggc ggcggcggcg gcggcggcgg 60

cggcggcggc ggcggcggcg gcggcgg 87

<210> 63
<211> 90
<212> DNA
<213> Artificial Sequence

<220>
<221> source
<223> /note="Description of Artificial Sequence: Synthetic oligonucleotide"

<400> 63

cggcggcggc ggcggcggcg gcggcggcgg aggcggcggc ggcggcggcg gcggcggcgg 60

aggcggcggc ggcggcggcg gcggcggcgg 90

<210> 64
<211> 120
<212> DNA
<213> Artificial Sequence

<220>
<221> source
<223> /note="Description of Artificial Sequence: Synthetic polynucleotide"

<400> 64

cggcggcggc ggcggcggcg gcggcggcgg cggaggcggc ggcggcggcg gcggcggcgg 60

cggcggcggc ggcggcggcg gcggcggcgg cggcggcggc ggcggcggcg gcggcggcgg 120

<210> 65
<211> 120
<212> DNA
<213> Artificial Sequence

<220>
<221> source
<223> /note="Description of Artificial Sequence: Synthetic polynucleotide"

<400> 65

cggcggcggc ggcggcggcg gcggcggcgg cggaggcggc ggcggcggcg gcggcggcgg 60

aggcggcggc ggcggcggcg gcggcggcgg cggcggcggc ggcggcggcg gcggcggcgg 120

<210> 66
<211> 12
<212> DNA
<213> Artificial Sequence

<220>
<221> source
<223> /note="Description of Artificial Sequence: Synthetic oligonucleotide"

<400> 66
ccccgccccg cg 12